# EUROPÄISCHE PATENTSCHRIFT

(11) **EP 2 614 727 B1**
(45) Veröffentlichungstag und Bekanntmachung des Hinweises auf die Patenterteilung: **07.09.2016**
(21) Anmeldenummer: 12150550.7
(22) Anmeldetag: 10.01.2012
(51) Int. Cl.: A23L 27/20, A23L 33/00, A23L 33/105, A23L 33/20, A61K 31/165, C07C 233/22

(54) **N-Nonanoylvanillylamin als Mittel zur Reduzierung des Appetits und als Mittel zur Vermittlung eines Gefühls von Sättigung sowie entsprechende oral konsumierbare Produkte und Verfahren**
N-Nonanoylvanillylamine as an appetite reduction agent and as a means for generating the feeling of being full and corresponding orally consumable products and method
N-nonanoylvanillylamine comme moyen de réduction de l'appétit, comme moyen de transmission d'une sensation de satiété ainsi que produits consommables oralement et procédé

(43) Veröffentlichungstag der Anmeldung: 17.07.2013
(73) Patentinhaber: Symrise AG, 37603 Holzminden (DE)
(72) Erfinder: Ley, Jakob, 37603 Holzminden (DE); Krammer, Gerhard, 37603 Holzminden (DE); Somoza, Veronika, 3400 Weidling (AT); Rohm, Barbara, 1090 Wien (AT); Somoza, Mark, 3400 Weidling (AT)
(74) Vertreter: Fabry, Bernd

(56) Entgegenhaltungen:
- WO-A2-2006/060505
- WO-A2-2006/086764
- WO-A2-2008/040361
- WO-A2-2011/045732
- WO-A2-2011/063817
- US-A1- 2006 292 254
- US-A1- 2009 311 401
- DATABASE WPI Week 201079 Thomson Scientific, London, GB; AN 2010-N86992 XP002673734, & WO 2010/128788 A2 (UNIV ULSAN FOUND IND COOP) 11. November 2010 (2010-11-11)

## Beschreibung

Die Erfindung betrifft primär die nicht-therapeutische Verwendung von N-Nonanoylvanillylamin enthalten in einem oral konsumierbaren Produkt, das kein Capsaicin enthält, als Mittel zur Reduzierung des Appetits und Mittel zur Vermittlung eines Gefühls von Sättigung.

Die Erfindung betrifft ferner ein Verfahren zur nicht-therapeutischen Reduzierung des Appetits und zur Vermittlung eines Gefühls von Sättigung. Ferner umfasst die Erfindung Stoffmischungen zur Anwendung in bestimmten nicht-therapeutischen Verfahren, umfassend N-Nonanoylvanillylamin und ein oder mehrerer weitere Stoffe, als Mittel zur Reduzierung des Appetits und Mittel zur Vermittlung eines Gefühls von Sättigung. Die Erfindung betrifft auch die nicht-therapeutische Verwendung entsprechender Stoffmischungen. Schließlich betrifft die Erfindung auch N-Nonanoylvanillylamin umfassende oral konsumierbare Produkte (insbesondere Lebensmittel und Futtermittel), wobei das N-Nonanoylvanillylamin in einer (a) den Appetit reduzierenden und (b) ein Gefühl von Sättigung bewirkenden, jedoch geringen Konzentration vorliegt.

Das durch mangelnde Bewegung und/oder zu hohe Nahrungsaufnahme verursachte häufige Auftreten von andauerndem Übergewicht kann zu chronischen Krankheitsbildern wie Fettleibigkeit, Insulinresistenz, Lipidstoffwechselstörungen und/oder Bluthochdruck, deren schwerwiegenden Folgeerkrankungen Diabetes Typ II, Arteriosklerose, Herz- oder Hirninfarkt und damit letztendlich zum verfrühten Tod führen. Insbesondere ein hoher Gehalt an leicht verstoffwechselbaren Kohlenhydraten, Proteinen und vor allem Fetten in der Nahrung führt zur Bildung von Fettdepots und kann schließlich zu den oben genannten Problemen stark beitragen. Um die Aufnahme dieser hedonisch oft sehr bevorzugten Nahrungsmittelbestandteile, vor allem der Fette und süßen Kohlenhydrate (Zucker), zu begrenzen, werden oft deren Gehalte in kalorienreduzierten Lebensmitteln, den sogenannten "Light-Produkten", stark gesenkt und durch Ersatzstoffe (Verdicker für Fette, nicht-kalorische Süßstoffe statt der Zucker) ausgetauscht.

Beim Konsum von "Light-Produkten" kann es bei einem Produkt, das aufgrund geschickter Formulierung einen vergleichbaren hedonischen Wert wie das hoch-energetische Originalprodukt besitzt, oft vorkommen, dass es in größeren Mengen konsumiert wird und es dann im ungünstigsten Fall sogar zu einer erhöhten Aufnahme kalorisch relevanter Nahrungsmittelbestandteile kommt und somit das Ziel verfehlt wird, die aufgenommene Kalorienmenge abzusenken.

Um einer erhöhten Aufnahme kalorisch relevanter Nahrungsmittelbestandteile entgegenzuwirken, besteht schon länger der Wunsch, Nahrungsbestandteile, insbesondere als sicher bewertete, bereits zugelassene und allgemein akzeptierte Aromastoffe zu finden, die das Hungergefühl und den natürlichen Appetit reduzieren und/oder das Sättigungsgefühl entsprechend erhöhen können. Es ist bekannt, dass durch Erhöhung der Dopamin-und der Serotonin-Ausschüttung in bestimmten Hirnarealen bei gleichzeitiger Exposition mit Nährstoffen durch die aufgenommenen oral konsumierbaren Produkte (insbesondere Lebensmittel) sowie durch Induktion von Leptin-Rezeptor- und Serotonin-Rezeptor-Proteinen der Appetit negativ und die Sättigung positiv beeinflusst werden können.

Eine Aufnahme von Serotonin und Dopamin über die Nahrung erhöht die Serotonin- bzw. Dopaminkonzentration im Blut und steht mit einiger Wahrscheinlichkeit auch für Wechselwirkungen mit Rezeptoren im Hirn zur Verfügung. Um die Serotonin- bzw. Dopaminkonzentration im Hirn zu erhöhen, ist es vorteilhaft, die Ausschüttung von Serotonin bzw. Dopamin im Hirn zu stimulieren.

In Untersuchungen hat sich gezeigt, dass reines Capsaicin, der wichtigste Scharfstoff aus der Chillischote (*Capsicum anuum*), einen Appetit-reduzierenden und Sättigungssteigernden Effekt zeigen kann (Smeets, A. J. P. G.; Westerterp-Plantenga, M., Capsaicin. In Weight Control and Slimming Ingredients in Food Technologies, Cho, Susan S. (Ed.), pp. 201-211, Wiley-Blackwell: Ames, Iowa, 2010.). Dieser beobachtete Effekt beruht vermutlich darauf, dass Capsaicin bei oraler Aufnahme ein Gefühl von Hitze vermittelt.

Der Einsatz von Capsaicin in Lebensmitteln ist allerdings in der Europäischen Union nicht erlaubt (wurde von der Community Flavoring List in 2004 gestrichen), da das genotoxische Potential der Verbindung negativ bewertet wurde (European Food Safety Authority (EFSA), P., Italy, Opinion of the Scientific Committee on Food on Capsaicin. *European Comission* 2002, (SDF/CS/FLAV/FLAVOUR/8 ADD1 Final)). Zudem ist der Einsatz in Lebensmitteln oft schwierig, da Capsaicin einen niedrigen Geschmacksschwellenwert und eine hohe Potenz als Scharfstoff (16,000,000 Scoville units, vgl. http://en.wikipedia.org/wiki/Capsaicin; Version des Eintrags mit letzten Änderungen vom 11. November 2011, 21:02 Uhr) hat. Capsaicin wird, auch auf Grund des hohen Preises des Reinstoffs, zudem nahezu ausschließlich in Form eines Capsicumextrakts verwendet, der neben weiteren Scharfstoffen noch Reste anderer, nach Capsicum schmeckender oder riechender Aromastoffe enthält und daher für den breiten Einsatz nur bedingt geeignet ist.

Es war die primäre Aufgabe der vorliegenden Erfindung, einen (vorzugsweise lebensmittelrechtlich zugelassenen) Aromastoff zu finden, der - in seiner Wirkung insoweit ähnlich zu Capsaicin - als Mittel zur Reduzierung des Appetits und als Mittel zur Vermittlung eines Gefühls von Sättigung verwendet werden kann.

Diese Aufgabe wird gelöst durch die nicht-therapeutische Verwendung von N-Nonanoylvanillylamin enthalten in einem oral konsumierbaren Produkt, das kein Capsaicin enthält, in einer Konzentration von 0,05 - 0,001 mg/Kg bezogen auf die Gesamtmasse des oral konsumierbaren Produktes
(a) als Mittel zur Reduzierung des Appetits, vorzugsweise zur Reduzierung der kalorischen Aufnahme und dabei bevorzugt zur nicht-therapeutischen Reduktion des Gewichtes,
   und
(b) als Mittel zur Vermittlung eines Gefühls von Sättigung,
   vorzugsweise zur Reduzierung der kalorischen Aufnahme und dabei bevorzugt zur nicht-therapeutischen Reduktion des Gewichtes.
N-Nonanoylvanillylamin ist ein bekannter und zugelassener Aromastoff (Flavis-Nummer 16.006; EFSA: ist in FGE.86 mit 'no safety concern' bewertet worden; CAS-Nummer 2444-46-4) und wird bereits in einer Vielzahl von Lebensmitteln zur Erzielung einer deutlichen Schärfe eingesetzt. Der Reizschwellenwert von N-Nonanoylvanillylamin liegt bei 0,054 mg/kg, der Erkennungsschwellenwert 0,107 mg/kg (vgl. Kollmannsberger, H. Inhaltsstoffzusammensetzung und sensorische Qualität von 20 Kultivaren verschiedener Capsicum-Arten. Dissertation, Technische Universität München, Weihenstephan, 2007, Seite 9). Typische Einsatzkonzentrationen in Lebensmitteln sind 1 -10 mg/kg und für Kaugummi 50 mg/kg (vgl. Flavor Base 2010, Eintrag "Nonanoyl hydroxymethoxybenzylamide", Record # 2174, Leffingwell & Associates). US 2006 1029 2254 beschreibt die verwendung von Vanillylamid aus verringerung des Appetits im Konzentrationsbereich von 1-1000ppm.

N-Nonanoylvanillylamin ist ebenfalls unter den Bezeichnungen Pseudocapsaicin, N-((4-Hydroxy- 3-methoxyphenyl) methyl)nonanamid, Pelargonsäure-Vanillylamid und Nonivamid bekannt. N-Nonanoylvanillylamin (Verbindung I) ist zur Verdeutlichung in der folgenden Abbildung dargestellt:

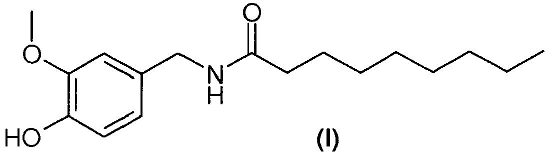

Wie vorstehend bereits am Beispiel des Capsaicins angemerkt, ist die Verwendung scharf schmeckender Verbindungen nicht immer problemlos möglich. Häufig ist eine Schärfe von oral konsumierbaren Produkten (insbesondere Lebensmittel, Futtermittel und Arzneimittel) nicht erwünscht und zu scharf schmeckende oral konsumierbare Produkte werden vom Konsumenten häufig abgelehnt. Es war deshalb eine zusätzliche Aufgabe der vorliegenden Erfindung eine Substanz zu finden, die die in der Aufgabenstellung aufgeführten Eigenschaften aufweist und so eingesetzt werden kann, dass sie die erfindungsgemäße Aufgabe löst und dabei die organoleptischen Eigenschaften eines oral konsumierbaren Produktes (insbesondere Lebensmittels und Futtermittels) nicht bzw. nicht signifikant beeinflusst und somit ohne sensorische bzw. sensorisch signifikante Beeinflussung eines oral konsumierbaren Produktes in diesem eingesetzt werden kann.

Überraschenderweise hat sich gezeigt, dass N-Nonanoylvanillylamin in der Lage ist, bereits
(a) in einem Konzentrationsbereich von 0,01 µM bis 10 µM (0,003 mg/kg bis 3 mg/kg) die Serotonin-Freisetzung in Neuronen um maximal bis zu ca. 270 % gegen die jeweilige Kontrolle anzuregen
   und
(b) in einer Konzentration von etwa 0,03 mg/kg (entspricht 0,1 µM) die Dopamin-Freisetzung nach Acetylcholin-Stimulierung um bis zu ca. 700 % gegen die jeweilige Kontrolle anzuregen,
also jeweils in einer Konzentration, die sowohl deutlich unterhalb der Schwellenwerte als auch weit unter typischen Einsatzkonzentrationen in oral konsumierbaren Produkten (insbesondere Lebensmitteln und Futtermitteln) liegt. Vgl. hierzu die Beispiele weiter unten.

Die Lipophilie des N-Nonanoylvanillylamins bedingt generell eine gute Bioverfügbarkeit, d.h. eine gute Resorption vom Magen-Darm-Trakt in die Blutlaufbahn. So treten insbesondere auch bei Einsatzkonzentrationen des N-Nonanoylvanillylamins unterhalb des geschmacklichen Erkennungsschwellenwerts, also unterhalb von 0,1 mg/kg, und bei Aufnahme üblicher Verzehrsmengen von oral konsumierbaren Produkten (insbesondere von Lebensmitteln, Futtermitteln und Arzneimitteln) die oben genannten biologischen Effekte *in vivo* ein.

Vorzugsweise wird N-Nonanoylvanillylamin zur erfindungsgemäßen Anwendung in einer erfindungsgemäßen nicht-therapeutischen Verwendung eingesetzt, wobei das N-Nonanoylvanillylamin als Bestandteil eines oral konsumierbaren Produktes (insbesondere eines Lebensmittels und Futtermittels) eingesetzt wird, wobei
- das N-Nonanoylvanillylamin in einer Konzentration von 0,05 mg/kg oder weniger, mindestens abu einer Konzentration von 0,001 mg/kg, vorliegt, bezogen auf die Gesamtmasse des oral konsumierbaren Produktes, und
- das oral konsumierbare Produkt kein Capsaicin umfasst, vorzugsweise kein Capsaicin und außer N-Nonanoylvanillylamin keine weiteren Capsaicinoide umfasst.

Bevorzugt ist ein erfindungsgemäß oral konsumierbares Produkt ausgewählt aus der Gruppe bestehend aus Lebensmitteln (insbesondere flüssige oder feste, einschließlich Halbfertigwaren) und Futtermitteln.

Im Rahmen des vorliegenden Textes umfasst der Begriff "Lebensmittel" eine Vielzahl von Produkten. Unter den Begriff Lebensmittel fallen insbesondere Produkte, wie sie weiter unten im Zusammenhang mit erfindungsgemäßen Lebensmitteln diskutiert werden.

Der Begriff "Lebensmittel" umfasst insbesondere Produkte, die gemäß der VERORDNUNG (EG) Nr. 178/2002 DES EUROPÄISCHEN PARLAMENTS UND DES RATES vom 28. Januar 2002 Lebensmittel sind. Gemäß dieser Verordnung sind "Lebensmittel" alle Stoffe oder Erzeugnisse, die dazu bestimmt sind oder von denen nach vernünftigem Ermessen erwartet werden kann, dass sie in verarbeitetem, teilweise verarbeitetem oder unverarbeitetem Zustand von Menschen aufgenommen werden. Zu "Lebensmitteln" zählen auch Getränke, Kaugummi sowie alle Stoffe - einschließlich Wasser -, die dem Lebensmittel bei seiner Herstellung oder Ver- oder Bearbeitung absichtlich zugesetzt werden.

Der Begriff "Futtermittel" umfasst im Rahmen des vorliegenden Textes alle Formen von Tiernahrung. Eine Vielzahl der nachstehend genannten Lebensmittel kann auch als Futtermittel eingesetzt werden.

Es versteht sich, dass Lebensmittel oder Futtermittel durch den Zusatz von Stoffen oder Stoffzusammensetzungen, die als Mittel mit Eigenschaften zur Heilung oder zur Verhütung menschlicher oder tierischer Krankheiten bestimmt sind, in entsprechende Arzneimittel überführt werden können.

Der Begriff "Arzneimittel" umfasst im Rahmen des vorliegenden Textes Stoffe oder Stoffzusammensetzungen, die als Mittel mit Eigenschaften zur Heilung oder zur Verhütung menschlicher oder tierischer Krankheiten bestimmt sind oder aber im oder am menschlichen oder tierischen Körper verwendet oder einem Menschen bzw. Tier verabreicht werden können, um entweder die menschlichen bzw. tierischen physiologischen Funktionen durch eine pharmakologische, immunologische oder metabolische Wirkung wiederherzustellen, zu korrigieren oder zu beeinflussen oder eine medizinische Diagnose zu erstellen. Arzneimittel können im Einzelfall zu nicht-therapeutischen, insbesondere kosmetischen Zwecken eingesetzt werden.

Die vorliegende Erfindung betrifft auch ein Verfahren
(a) zur nicht-therapeutischen Reduzierung des Appetits,
   vorzugsweise zur nicht-therapeutischen Reduzierung der kalorischen Aufnahme und dabei bevorzugt zur nicht-therapeutischen Reduktion des Gewichtes,
   und
(b) zur nicht-therapeutischen Vermittlung eines Gefühls von Sättigung,
   vorzugsweise zur nicht-therapeutischen Reduzierung der kalorischen Aufnahme und dabei bevorzugt zur nicht-therapeutischen Reduktion des Gewichtes, mit folgendem Schritt:
   - Verabreichen von N-Nonanoylvanillylamin enthalten in einem oral konsumierbaren Produkt, das kein Capsaicin enthält, in einer Konzentration von 0,05-0,001 mg/Kg bezogen auf die Gesamtmasse des oral konsumierbaren Produktes an ein menschliches oder tierisches Individuum.

Die vorliegende Erfindung betrifft ebenfalls eine nicht-therapeutische Verwendung einer Stoffmischung, umfassend N-Nonanoylvanillylamin und einen oder mehrere weitere Stoffe,
(a) als Mittel zur Reduzierung des Appetits,
   vorzugsweise zur Reduzierung der kalorischen Aufnahme und dabei bevorzugt zur nicht-therapeutischen Reduktion des Gewichtes,
   und
(b) als Mittel zur Vermittlung eines Gefühls von Sättigung,
   vorzugsweise zur Reduzierung der kalorischen Aufnahme und dabei bevorzugt zur nicht-therapeutischen Reduktion des Gewichtes,
wobei das in der Stoffmischung enthaltene N-Nonanoylvanillylamin so eingesetzt wird, dass es den Appetit reduziert und ein Gefühl von Sättigung hervorruft, nämlich gemäß Anspruch 1. In einer bevorzugten erfindungsgemäßen Stoffmischung zur Anwendung und in einer bevorzugten erfindungsgemäßen Verwendung einer Stoffmischung ist die Stoffmischung jeweils ein oral konsumierbares Produkt (insbesondere Lebensmittel oder Futtermittel) oder Bestandteil eines oral konsumierbaren Produktes (insbesondere Lebensmittels, Futtermittels),
wobei das oral konsumierbare Produkt das N-Nonanoylvanillylamin in einer Konzentration von weniger als 0,05 mg/kg und mehr als 0,001 mg/kg umfasst, bezogen auf die Gesamtmasse des oral konsumierbaren Produktes,
wobei die Stoffmischung kein Capsaicin umfasst, und außer N-Nonanoylvanillylamin keine weiteren Capsaicinoide umfasst.

In einer besonders bevorzugten erfindungsgemäßen Stoffmischung zur Anwendung und in besonders bevorzugten erfindungsgemäßen nicht-therapeutischen Verwendungen einer Stoffmischung, handelt es sich bei den weiteren Stoffen um vorzugsweise sprühgetrocknete Stoffe, die zum Verzehr geeignete Bestandteile, feste Trägerstoffe und ggf. bestimmte Aromastoffe bzw. Aromakompositionen umfassen.

In weiteren besonders bevorzugten erfindungsgemäßen Stoffmischungen zur Anwendung und in besonders bevorzugten erfindungsgemäßen Verwendungen einer Stoffmischung, handelt es sich bei den weiteren Stoffen um zum Verzehr geeignete feste Trägerstoffe.

Vorteilhafte Trägerstoffe in diesen bevorzugten erfindungsgemäßen (vorzugsweise sprühgetrockneten) Stoffmischungen sind Siliciumdioxid (Kieselsäure, Kieselgel), Kohlenhydrate und/oder Kohlenhydratpolymere (Polysaccharide), Cyclodextrine, Stärken, abgebaute Stärken (Stärkehydrolysate), chemisch oder physikalisch modifizierte Stärken, modifizierte Cellulosen, Gummi Arabicum, Ghatti-Gummi, Traganth, Karaya, Carrageenan, Guarkernmehl, Johannisbrotkernmehl, Alginate, Pektin, Inulin oder Xanthan Gum zu nennen. Bevorzugte Stärkehydrolysate sind Maltodextrine und Dextrine.

Bevorzugte Trägerstoffe sind Siliciumdioxid, Gummi Arabicum und Maltodextrine, wobei hier wiederum Maltodextrine mit DE-Werten im Bereich 5 bis 20 bevorzugt sind. Es ist dabei unerheblich, welche Pflanze ursprünglich die Stärke zur Herstellung der Stärkehydrolysate geliefert hat. Geeignet und leicht verfügbar sind Maisbasierende Stärken sowie Stärken aus Tapioka, Reis, Weizen oder Kartoffeln. Die Trägerstoffe können dabei auch als Fließhilfsmittel fungieren, wie beispielsweise Siliciumdioxid.

Die erfindungsgemäßen Stoffmischungen, die neben dem erfindungsgemäß zu verwendenden N-Nonanoylvanillylamin noch einen oder mehrere feste Trägerstoffe umfassen, können beispielsweise durch mechanische Mischvorgänge, wobei gleichzeitig auch eine Zerkleinerung der Partikel erfolgen kann, oder mittels Sprühtrocknung hergestellt werden. Bevorzugt sind erfindungsgemäße Zusammensetzungen, die feste Trägerstoffe umfassen und mittels Sprühtrocknung hergestellt sind; hinsichtlich der Sprühtrocknung wird verwiesen auf US 3,159,585, US 3,971,852, US 4,532,145 oder US 5,124,162.

Bevorzugte erfindungsgemäße, Trägerstoffe umfassende Stoffmischungen, die mittels Sprühtrocknung hergestellt wurden, besitzen eine mittlere Partikelgröße im Bereich von 30 bis 300 µm und eine Restfeuchte von kleiner oder gleich 5 Gew.-%.

Eine erfindungsgemäße Stoffmischung gemäß Anspruch 3 ist vorzugsweise eine (vorzugsweise sprühgetrocknete) Stoffmischung, die neben
(i) N-Nonanoylvanillylamin in einer Konzentration von 0,05 - 0,001 mg/Kg bezogen auf die Gesamtmasse der Stoffmischung und
(ii) festen Trägerstoffen
   zusätzlich
(iii) einen oder mehrere flüchtige Aromastoffe,
umfasst bzw. aus den genannten Komponenten besteht. Dabei gilt hinsichtlich bevorzugter Verbindungen und Mischungen das oben Gesagte entsprechend. Die Stoffmischung umfasst kein Capsaicin und außer N-Nonanoylvanillylamin keine weiteren Capsaicinoide.

Der oder zumindest einer der flüchtigen Aromastoffe der Komponente (iii) ist dabei vorzugsweise eine sensorisch wirksame Komponente mit einem Dampfdruck von größer oder gleich 0,01 Pa bei 25°C, vorzugsweise einem Da mpfdruck von größer oder gleich 0,025 Pa bei 25°C, und er wird bevorzugt eingesetzt in einer Konzentration, die größer ist als sein Reizschwellenwert. Eine Vielzahl der flüchtigen Aromastoffe weist einen Dampfdruck von größer oder gleich 1 Pa bei 25°C auf; die se Aromastoffe werden für die Verwendung in erfindungsgemäßen Stoffmischungen als bevorzugt angesehen.

Beispiele für Aromastoffe der Komponente (iii), die Bestandteil der Stoffmischung sein können, finden sich z.B. in H. Surburg, J. Panten, Common Fragrance and Flavor Materials, 5th. Ed., Wiley-VCH, Weinheim 2006.

Aromastoffe der Komponente (iii) können in Form von Aromakompositionen eingesetzt werden, die wiederum in Form von Reaktionsaromen (Maillard-Produkte) und/oder Extrakten bzw. ätherischen Ölen von Pflanzen oder Pflanzenteilen bzw. Fraktionen davon eingesetzt werden können, wobei Capsicum-Spezies ausgeschlossen sind.

In einem weiteren Aspekt betrifft die vorliegende Erfindung ein oral konsumierbares Produkt (insbesondere Lebensmittel und Futtermittel), umfassend N-Nonanoylvanillylamin und einen oder mehrere weitere Stoffe, wobei
das N-Nonanoylvanillylamin
- in einer den Appetit reduzierenden und ein Gefühl von Sättigung bewirkenden Konzentration
   und gleichzeitig
- in einer Konzentration von 0,05 mg/kg oder weniger, bezogen auf die Gesamtmasse des oral konsumierbaren Produktes vorliegt
und gleichzeitig
- in einer Konzentration von zumindest 0,001 mg/kg oder mehr, bezogen auf die Gesamtmasse des oral konsumierbaren Produktes, vorzugsweise in einer Konzentration von 0,005 mg/kg oder mehr, ganz besonders bevorzugt in einer Konzentration von 0,01 mg/kg oder mehr vorliegt und
das oral konsumierbare Produkt kein Capsaicin umfasst, und außer N-Nonanoylvanillylamin keine weiteren Capsaicinoide umfasst.
Besonders bevorzugt ist ein erfindungsgemäßes oral konsumierbares Produkt, bei dem es sich um ein Lebensmittel; und Futtermittel handelt.

Um eine Reduktion des Körpergewichtes beim Konsumenten zu unterstützen, hat es sich als sinnvoll erwiesen, die kalorische Aufnahme zu begrenzen. Dies kann zum einen dadurch erfolgen, dass die erfindungsgemäße Verwendung von N-Nonanoylvanillylamin in oral konsumierbaren Produkten (insbesondere Lebensmitteln und Futtermitteln) ein Gefühl von Sättigung vermittelt und gleichzeitig den Appetit reduziert. Dadurch wird der Konsument dazu veranlasst, den Verzehr von energiehaltigen Produkten zu begrenzen. Die kalorische Aufnahme lässt sich zusätzlich reduzieren, wenn erfindungsgemäße oral konsumierbare Produkte (insbesondere Lebensmittel und Futtermittel) zum Verzehr angeboten werden, die ihrerseits bereits eine geringe Energiedichte aufweisen.

Ein bevorzugtes erfindungsgemäßes oral konsumierbares Produkt (insbesondere Lebensmittel und Futtermittel) enthält nicht mehr als 200 kcal/100g des oral konsumierbaren Produktes, bevorzugt nicht mehr als 100 kcal/100g, besonders bevorzugt nicht mehr als 40 kcal/100g.

Bevorzugte erfindungsgemäße oral konsumierbare Produkte (insbesondere Lebensmittel oder Futtermittel) sind jegliche zum Verzehr geeignete der Ernährung, der Mundpflege oder dem Genuss dienende Zubereitungen oder Zusammensetzungen und sind regelmäßig Produkte, die dazu bestimmt sind, in die menschliche bzw. tierische Mundhöhle eingebracht zu werden, dort eine bestimmte Zeit zu verbleiben und anschließend entweder verzehrt (z.B. verzehrfertige Lebensmittel oder Futtermittel, siehe auch weiter unten) oder wieder aus der Mundhöhle entfernt zu werden (z.B. Kaugummis oder Mundpflegeprodukte oder medizinische Mundspülungen). Zu diesen Produkten gehören dabei sämtliche Stoffe oder Erzeugnisse, die dazu bestimmt sind, in verarbeitetem, teilweise verarbeitetem oder unverarbeitetem Zustand vom Menschen oder Tier aufgenommen zu werden. Hierzu zählen auch Stoffe, die oral konsumierbaren Produkten (insbesondere Lebensmittel oder Futtermittel) bei ihrer Herstellung, Verarbeitung oder Bearbeitung zugesetzt werden und dazu vorgesehen sind, in die menschliche oder tierische Mundhöhle eingebracht zu werden.

Zu den erfindungsgemäßen oral konsumierbaren Produkten (insbesondere Lebensmittel oder Futtermittel) zählen auch Stoffe, die dazu bestimmt sind, in unverändertem, zubereitetem oder verarbeitetem Zustand vom Menschen oder Tier geschluckt und dann verdaut zu werden; zu den erfindungsgemäßen oral konsumierbaren Produkten gehören insoweit auch Umhüllungen, Überzüge oder sonstige Umschließungen, die dazu bestimmt sind, mit verschluckt zu werden, oder bei denen ein Verschlucken vorauszusehen ist. Der Begriff "oral konsumierbares Produkt" umfasst verzehrfertige Lebensmittel und Futtermittel, d.h. Lebensmittel oder Futtermittel, die hinsichtlich der für den Geschmack maßgeblichen Substanzen bereits vollständig zusammengesetzt sind. Unter den Begriffen "verzehrfertiges Lebensmittel" oder "verzehrfertiges Futtermittel" fallen auch Getränke sowie feste oder halbfeste verzehrfertige Lebensmittel oder Futtermittel. Als Beispiele seien Tiefkühlprodukte genannt, die vor dem Verzehr aufgetaut und auf Verzehrtemperatur erwärmt werden müssen. Auch Produkte wie Joghurt oder Eiscreme aber auch Kaugummis oder Hartkaramellen zählen zu den verzehrfertigen Lebensmitteln oder Futtermitteln.

Bevorzugte erfindungsgemäße oral konsumierbare Produkte (insbesondere Lebensmitteln und Futtermitteln) umfassen auch "Halbfertigwaren". Unter einer Halbfertigware ist im Zusammenhang des vorliegenden Textes ein oral konsumierbares Produkt zu verstehen, welches aufgrund eines sehr hohen Gehaltes an Aroma- und Geschmacksstoffen für die Verwendung als verzehrfertiges oral konsumierbares Produkt (insbesondere Lebensmitteln oder Futtermitteln) ungeeignet ist. Erst durch Mischen mit mindestens einem weiteren Bestandteil (d.h. durch Verringern der Konzentration der betreffenden Aroma- und Geschmacksstoffe) und gegebenenfalls weitere Prozessschritte (z.B. Erwärmen, Gefrieren) wird die Halbfertigware in ein verzehrfertiges oral konsumierbares Produkt (insbesondere Lebensmittel oder Futtermittel) überführt. Als Beispiel für Halbfertigwaren seien hier Tütensuppen, Backaromen und Puddingpulver genannt.

Zu erfindungsgemäßen oral konsumierbaren Produkten (insbesondere Lebensmitteln oder Futtermitteln) zählen auch "Mundpflegeprodukte". Unter einem Mundpflegeprodukt (auch Mundhygieneprodukt oder mundhygienische Zubereitung genannt) im Sinne der Erfindung wird eine der dem Fachmann geläufigen Formulierungen zur Reinigung und Pflege der Mundhöhle und des Rachenraumes sowie zur Erfrischung des Atems verstanden. Hierbei ist die Pflege der Zähne und des Zahnfleisches ausdrücklich eingeschlossen. Darreichungsformen gebräuchlicher mundhygienischer Formulierungen sind insbesondere Cremes, Gele, Pasten, Schäume, Emulsionen, Suspensionen, Aerosole, Sprays, wie auch Kapseln, Granulate, Pastillen, Tabletten, Bonbons oder Kaugummis, ohne dass diese Aufzählung für die Zwecke dieser Erfindung limitierend verstanden werden soll.

Bevorzugt umfasst ein erfindungsgemäßes oral konsumierbares Produkt (insbesondere Lebensmittel oder Futtermittel), eine oder mehrere der Ernährung oder dem Genuss dienende Zubereitungen. Hierunter fallen insbesondere, Süßwaren (z.B. Schokoladen, Schokoladenriegelprodukte, sonstige Riegelprodukte, Fruchtgummi, Dragees, Hart- und Weichkaramellen, Kaugummi), nicht-alkoholische Getränke (z.B. Kakao, Kaffee, Grüntee, Schwarztee, mit (Grün-, Schwarz-)Tee-Extrakten angereicherte (Grün-, Schwarz-)Tee-Getränke, Rooibos-Tee, andere Kräutertees, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Getreideprodukte (z.B. Frühstückscerealien, Müsliriegel, vorgegarte Fertigreis-Produkte), Milchprodukte (z.B. Vollfett- oder fettreduzierte oder fettfreie Milchgetränke, Milchreis, Joghurt, Kefir, Frischkäse, Weichkäse, Hartkäse, Trockenmilchpulver, Molke, Butter, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, Getränke, enthaltend isoliertes oder enzymatisch behandeltes Sojaprotein, Sojamehl enthaltende Getränke, Sojalecithin-haltige Zubereitungen, fermentierte Produkte wie Tofu oder Tempe oder daraus gefertigte Produkte und Mischungen mit Fruchtzubereitungen und fakultativ Aromen), Süßstoffzubereitungen,-tabletten oder -sachets, sonstige Zubereitungen zum Süßen oder Weißen von Getränken oder anderen Nahrungsmitteln. Die Zubereitungen im Sinne der Erfindung können auch als Halbfertigware zur Herstellung weiterer der Ernährung oder dem Genuss dienenden Zubereitungen dienen. Die Zubereitungen im Sinne der Erfindung können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen und als Nahrungsergänzungsmittel vorliegen.

Besonders bevorzugt sind kalorienreduzierte Süßwaren (z.B. Müsliriegelprodukte, Fruchtgummi, Dragees, Hart- und Weichkaramellen, Kaugummi), nicht-alkoholische Getränke (z.B. Kakao, Grüntee, Schwarztee, mit (Grün-, Schwarz-)Tee-Extrakten angereicherte (Grün-, Schwarz-)Tee-Getränke, Rooibos-Tee, andere Kräutertees, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke), Getreideprodukte (z.B. Frühstückscerealien, Müsliriegel, vorgegarte Fertigreis-Produkte), Milchprodukte (z.B. Vollfett- oder fettreduzierte oder fettfreie Milchgetränke, Milchreis, Joghurt, Kefir, Trockenmilchpulver, Molke, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, Getränke, enthaltend isoliertes oder enzymatisch behandeltes Sojaprotein, Sojamehl enthaltende Getränke, Sojalecithin-haltige Zubereitungen, fermentierte Produkte wie Tofu oder Tempe oder daraus gefertigte Produkte und Mischungen mit Fruchtzubereitungen und fakultativ Aromen), Süßstoffzubereitungen, -tabletten oder -sachets.

Insbesondere bevorzugt sind kalorienreduzierte oder kalorienfreie Süßwaren (z.B. Müsliriegelprodukte, Fruchtgummi, Dragees, Hartkaramellen, Kaugummi), nicht-alkoholische Getränke (z.B. Grüntee, Schwarztee, mit (Grün-, Schwarz-)Tee-Extrakten angereicherte (Grün-, Schwarz-)Tee-Getränke, Rooibos-Tee, andere Kräutertees, fruchthaltige zuckerarme oder -freie Limonaden, isotonische Getränke, Nektare, Obst- und Gemüsesäfte, Frucht- oder Gemüsesaftzubereitungen), Instantgetränke (z.B. Instant-(Grün-, Schwarz, Roibos, Kräuter)Tee-Getränke), Getreideprodukte (z.B. zuckerarme oder -freie Frühstückscerealien, Müsliriegel), Milchprodukte (z.B. fettreduzierte oder fettfreie Milchgetränke, Joghurt, Kefir, Molke, Buttermilch), Produkte aus Sojaprotein oder anderen Sojabohnen-Fraktionen (z.B. Sojamilch und daraus gefertigte Produkte, Getränke, enthaltend isoliertes oder enzymatisch behandeltes Sojaprotein, Sojamehl enthaltende Getränke, Sojalecithin-haltige Zubereitungen, oder daraus gefertigte Produkte und Mischungen mit Fruchtzubereitungen und fakultativ Aromen) oder Süßstoffzubereitungen, -tabletten oder - sachets.

Die Zubereitungen können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen vorliegen, z. B. als Nahrungsergänzungsmittel.

Die Halbfertigwaren dienen in der Regel zur Herstellung von der Ernährung oder dem Genuss dienenden gebrauchs- oder verzehrfertigen Zubereitungen.

Weitere Bestandteile einer der Ernährung oder dem Genuss dienenden verzehrfertigen Zubereitung bzw. Halbfertigware können übliche Grund-, Hilfs- und Zusatzstoffe für Nahrungs- oder Genussmittel sein, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Kräuter, Nüsse, Gemüsesäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. γ-Aminobuttersäure, Taurin), Peptide (z.B. Glutathion), native oder prozessierte Proteine (z.B. Gelatine), Enzyme (z.B. Peptidasen), Nuclein-säuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (z.B. Inositolphosphat, Nucleotide wie Guanosinmonophosphat, Adenosin-monophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carrageenan, Alginat), Konservierungsstoffe (z.B. Benzoesäure und deren Salze, Sorbinsäure und deren Salze), Antioxidantien (z.B. Tocopherol, Ascorbinsäure), Chelatoren (z.B. Citronensäure), organische oder anorganische Säuerungsmittel (z.B. Essigsäure, Phosphorsäure), zusätzliche Bitterstoffe (z.B. Chinin, Coffein, Limonin, Amarogentin, Humolone, Lupolone, Catechine, Tannine), die enzymatische Bräunung verhindernde Stoffe (z.B. Sulfit, Ascorbinsäure), ethe-rische Öle, Pflanzenextrakte, natürliche oder synthetische Farbstoffe oder Farbpig-mente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigenzien.

Bevorzugt enthalten erfindungsgemäße oral konsumierbare Produkte (insbesondere Lebensmittel oder Futtermittel), z.B. solche in Form von Zubereitungen oder Halbfertigwaren, eine Aromakomposition, um den Geschmack und/oder den Geruch abzurunden und zu verfeinern. Eine Zubereitung kann als Bestandteile einen festen Trägerstoff und eine Aromakomposition umfassen. Geeignete Aromakompositionen enthalten z.B. synthetische, natürliche oder naturidentische Aroma-, Riech- und Geschmacksstoffe, Reaktionsaromen, Raucharomen oder andere aromagebende Zubereitungen (z.B. Protein[teil]hydrolysate, bevorzugt Protein[teil]hydrolysate mit hohem Arginin-Gehalt, Grillaromen, Pflanzenextrakte, Gewürze, Gewürzzubereitungen, Gemüse und/oder Gemüsezubereitungen) sowie geeignete Hilfs- und Trägerstoffe. Insbesondere sind hier die Aromenkompositionen oder deren Bestandteile geeignet, die einen röstigen, fleischigen (insbesondere Huhn, Fisch, Meerestiere, Rind, Schwein, Lamm, Schaf, Ziege), gemüsigen (insbesondere Tomate, Zwiebel, Knoblauch, Sellerie, Lauch, Pilze, Auberginen, Seetang), einen würzigen (insbesondere schwarzer und weißer Pfeffer, Kardamom, Muskat, Piment, Senf und Senf-Produkte), gebratenen, hefigen, gesotteen, fettigen, salzigen und/oder scharfen Aromaeindruck verursachen und somit den würzigen Eindruck verstärken können. In der Regel enthalten die Aromakompositionen mehr als einen der genannten Inhaltsstoffe.

Die Energiedichte eines oral konsumierbaren Produktes (insbesondere Lebensmitteln oder Futtermitteln) lässt sich senken, indem energiereiche Inhaltsstoffe des oral konsumierbaren Produktes gegen Ersatzstoffe (z.B. kalorienarme Verdickungsmittel statt Fette, kalorienarme oder kalorienfreie Süßstoffe statt üblicher Zucker) ausgetauscht werden. Der bereits oben diskutierte Nachteil, dass der Konsument ein oral konsumierbares Produkt (insbesondere ein Lebensmittel) mit reduzierter Energiedichte in größeren Mengen konsumiert und es dann im ungünstigsten Fall sogar zu einer erhöhten Aufnahme kalorisch relevanter Nahrungsmittelbestandteile kommt, wird durch die Verwendung von N-Nonanoylvanillylamin in oral konsumierbaren Lebensmitteln (insbesondre in Lebensmitteln) entgegengewirkt. In einem oral konsumierbaren Produkt enthaltenes N-Nonanoylvanillylamin vermittelt ein vorzeitiges Gefühl von Sättigung und reduziert gleichzeitig den Appetit.

Vorzugsweise ist ein erfindungsgemäßes oral konsumierbares Produkt ausgewählt aus der Gruppe bestehend aus
- Süßwaren, vorzugsweise kalorienreduzierte bzw. kalorienfreie Süßwaren, vorzugsweise ausgewählt aus der Gruppe bestehend aus Müsliriegelprodukte, Fruchtgummi, Dragees, Hartkramellen und Kaugummi,
- nicht-alkoholische Getränke, vorzugsweise ausgewählt aus der Gruppe bestehend aus Grüntee, Schwarztee, mit (Grün-, Schwarz-)Tee-Extrakten angereicherte (Grün-, Schwarz-)Tee-Getränke, Rooibos-Tee, andere Kräutertees, fruchthaltige zuckerarme oder -freie Limonaden, isotonische Getränke, Nektare, Obst- und Gemüsesäfte, Frucht- und Gemüsesaftzubereitungen,
- Instantgetränke, vorzugsweise ausgewählt aus der Gruppe bestehend aus Instant-(Grün-, Schwarz-, Rooibos-, Käuter-)Tee-Getränke,
- Getreideprodukte, vorzugsweise ausgewählt aus der Gruppe bestehend aus zuckerarmen und -freien Frühstückscerealien und Müsliriegeln,
- Milchprodukte, vorzugsweise ausgewählt aus der Gruppe bestehend aus fettreduzierten und fettfreien Milchgetränken, Joghurt, Kefir, Molke, Buttermilch und Eiscreme,
- Produkte aus Sojaprotein oder anderen Sojabohnen-Fraktionen, vorzugsweise ausgewählt aus der Gruppe bestehend aus Sojamilch, aus Sojamilch gefertigten Produkten, Getränken enthaltend isoliertes oder enzymatisch behandeltes Sojaportein, Sojamehl enthaltenden Getränken, Sojalecithin-haltigen Zubereitungen, aus Sojalecithin-haltigen Zubereitungen gefertigten Produkten und Mischungen mit Fruchtzubereitungen und ggf. Aromen,
- Süßstoffzubereitungen, -tabletten und -sachets
- Zuckerfreie Dragees,
- Eiscreme, mit oder ohne Bestandteile auf Basis von Milch, vorzugsweise zuckerfrei.

Bevorzugt enthält ein erfindungsgemäßes oral konsumierbares Produkt (insbesondere Lebensmittel oder Futtermittel) (a) einen, zwei oder mehrere Süßstoffe und/oder (b) ein, zwei oder mehrere Verdickungsmittel.

Der Begriff "Süßstoffe" bezeichnet hierbei Stoffe mit einer relativen Süßkraft von zumindest 25, bezogen auf die Süßkraft von Saccharose (welches somit die Süßkraft 1 hat). Vorzugsweise sind in einem erfindungsgemäßen oral konsumierbaren Produkt (insbesondere Lebensmittel und Futtermittel) (a) einzusetzende Süßstoffe nicht-kariogen und/oder besitzen einen Energiegehalt von maximal 5 kcal pro Gramm des oral konsumierbaren Produktes.

Vorteilhafte Süßstoffe in einem bevorzugten erfindungsgemäßen oral konsumierbaren Produkt (insbesondere Lebensmittel oder Futtermittel) sind ausgewählt aus den folgenden Gruppen (a1) und (a2):
(a1) natürlich vorkommende Süßstoffe, vorzugsweise ausgewählt aus der Gruppe bestehend aus
(a1-1) Miraculin, Monellin, Mabinlin, Thaumatin, Curculin, Brazzein, Pentaidin, D-Phenylalanin, D-Tryptophan, und aus natürlichen Quellen gewonnene Extrakte oder Fraktionen, enthaltend diese Aminosäuren und/oder Proteine, und den physiologisch akzeptablen Salzen dieser Aminosäuren und/oder Proteine, insbesondere den Natrium-, Kalium-, Calcium oder Ammoniumsalzen;
(a1-2) Neohesperidindihydrochalkon, Naringindihydrochalkon, Steviosid, Steviolbiosid, Rebaudiosiden, insbesondere Rebaudiosid A, Rebaudiosid B, Rebaudiosid C, Rebaudiosid D, Rebaudiosid E, Rebaudiosid F, Rebaudiosid G, Rebaudiosid H, Dulcosiden und Rubusosid, Suavioside A, Suavioside B, Suavioside G, Suavioside H, Suavioside I, Suavioside J, Baiyunoside 1 Baiyunoside 2, Phlomisoside 1, Phlomisoside 2, Phlomisoside 3, sowie Phlomisoside 4, Abrusoside A, Abrusoside B, Abrusoside C, Abrusoside D, Cyclocaryoside A und Cyclocaryoside I, Oslandin, Polypodosid A, Strogin 1, Strogin 2, Strogin 4, Selligueanin A, Dihydroquercetin-3-acetat, Perillartin, Telosmosid A₁₅, Periandrin I-V, Pterocaryosiden, Cyclocaryosiden, Mukuroziosiden, trans-Anethol, trans-Cinnamaldehyd, Bryosiden, Bryonosiden, Bryonodulcosiden, Carnosiflosiden, Scandenosiden, Gypenosiden, Trilobatin, Phloridzin, Dihydroflavanolen, Hematoxylin, Cyanin, Chlorogensäure, Albiziasaponin, Telosmosiden, Gaudichaudiosid, Mogrosiden, Mogrosid V, Hernandulcinen, Monatin, Phyllodulcin, Glycyrrhetinsäure und deren Derivaten, insbesondere deren Glycosiden wie Glycyrrhizin, und den physiologisch akzeptablen Salzen dieser Verbindungen, insbesondere den Natrium-, Kalium-, Calcium oder Ammoniumsalzen;
(a1-3) Extrakte oder angereicherte Fraktionen der Extrakte, ausgewählt aus der Gruppe bestehend aus Thaumatococcus-Extrakten (Katemfestaude), Extrakten aus *Stevia* ssp. (insbesondere *Stevia rebaudiana*), Swingle-Extrakten (*Momordica* bzw. *Siratia grosvenorii,* Luo-Han-Guo), Extrakten aus *Glycerrhyzia* ssp. (insbesondere *Glycerrhyzia glabra),* Extrakten aus *Rubus* ssp. (insbesondere *Rubus suavissimus*), Citrus-Extrakten und Extrakten aus *Lippia dulcis;*
(a2) synthetische süß schmeckende Stoffe, vorzugsweise ausgewählt aus der Gruppe bestehend aus Magap, Natriumcyclamat oder anderen physiologisch akzeptablen Salzen der Cyclamsäure, Acesulfam K oder anderen physiologisch akzeptablen Salzen des Acesulfam, Neohesperidindihydrochalkon, Naringindihydrochalkon, Saccharin, Saccharin-Natriumsalz, Aspartam, Superaspartam, Neotam, Alitam, Advantam, Perillartin, Sucralose, Lugduname, Carrelame, Sucrononate und Sucrooctate.

Vorteilhafte Verdickungsmittel in einem bevorzugten erfindungsgemäßen oral konsumierbaren Produkt (insbesondere Lebensmittel oder Futtermittel) sind ausgewählt aus der Gruppe bestehend aus: vernetzte Polyacrylsäuren und deren Derivate, Polysaccharide und deren Derivate, wie Xanthangummi, Agar-Agar, Alginate oder Tylosen, Cellulosederivate, z. B. Carboxymethylcellulose oder Hydroxycarboxymethylcellulose, Fettalkohole, Monoglyceride und Fettsäuren, Polyvinylalkohol und Polyvinylpyrrolidon.

Erfindungsgemäß bevorzugt ist ein oral konsumierbares Produkt (insbesondere Lebensmittel oder Futtermittel), das durch Milchsäurebakterien verdickte Milch und/oder durch Milchsäurebakterien verdickte Sahne enthält und vorzugsweise
ausgewählt ist aus der Gruppe bestehend aus oral konsumierbaren Produkten mit einem Fettgehalt von 4,0 Gew.-% oder weniger, bevorzugt von 1,5 Gew.-% oder weniger, besonders bevorzugt 0,5 Gew.-% oder weniger, jeweils bezogen auf das Gesamtgewicht des oral konsumierbaren Produktes,
und/oder
ausgewählt ist aus der Gruppe bestehend aus Joghurt, Kefir und Quark.

Vorzugsweise enthält das erfindungsgemäße oral konsumierbare Produkt (insbesondere Lebensmittel oder Futtermittel), das durch Milchsäurebakterien verdickte Milch und/oder durch Milchsäurebakterien verdickte Sahne enthält, einen Energiegehalt von nicht mehr als 150 kcal/100g des oral konsumierbaren Produktes, bevorzugt nicht mehr als 100 kcal/100g, besonders bevorzugt nicht mehr als 75 kcal/100g, besonders bevorzugt nicht mehr als 50 kcal/100g.

Ein bevorzugtes erfindungsgemäßes oral konsumierbares Produkt (insbesondere Lebensmittel oder Futtermittel), das durch Milchsäurebakterien verdickte Milch und/oder durch Milchsäurebakterien verdickte Sahne enthält, umfasst zusätzlich Früchte und/oder Fruchtzubereitungen.

Besonders bevorzugt ist ein erfindungsgemäßes oral konsumierbares Produkt (insbesondere Lebensmittel oder Futtermittel), das durch Milchsäurebakterien verdickte Milch und/oder durch Milchsäurebakterien verdickte Sahne enthält, wobei das oral konsumierbare Produkt (i) Zucker und/oder (ii) Verdickungsmittel und/oder (iii) Geliermittel und/oder (iv) Süßungsmittel und/oder (v) Aromen und/oder (vi) Konservierungsstoffe enthält.

"Zucker" ist im Rahmen des vorliegenden Textes (sofern nicht anders angegeben oder aus dem Kontext anders ersichtlich) der Sammelbegriff für alle süß schmeckenden Saccharide (Einfach- und Doppelzucker).

Vorteilhafterweise ist ein erfindungsgemäßes oral konsumierbares Produkt (insbesondere Lebensmittel oder Futtermittel), das durch Milchsäurebakterien verdickte Milch und/oder durch Milchsäurebakterien verdickte Sahne enthält, ein oral konsumierbares Produkt, das ein Probiotikum enthält, wobei das Probiotikum vorzugsweise ausgewählt ist aus der Gruppe bestehend aus Bifidobacterium animalis subsp. lactis BB-12, Bifidobacterium animalis subsp. lactis DN-173 010, Bifidobacterium animalis subsp. lactis HN019, Lactobacillus acidophilus LA5, Lactobacillus acidophilus NCFM, Lactobacillus johnsonii La1, Lactobacillus casei immunitass/defensis, Lactobacillus casei Shirota (DSM 20312), Lactobacillus casei CRL431, Lactobacillus reuteri (ATCC 55730) und Lactobacillus rhamnosus (ATCC 53013).

Besonders bevorzugt ist ein erfindungsgemäßes oral konsumierbares Produkt (insbesondere Lebensmittel oder Futtermittel), das ein Kaugummi ist und eine Kaugummibase enthält. Die Kaugummibase ist dabei vorzugsweise ausgewählt aus der Gruppe bestehend aus "chewing gum" - oder "bubble gum" - Basen. Letztere sind weicher, damit sich damit auch Kaugummiblasen bilden lassen. Erfindungsgemäß bevorzugte Kaugummibasen umfassen neben traditionell eingesetzten natürlichen Harzen oder dem Naturlatex Chicle Elastomere wie Polyvinylacetate (PVA), Polyethylene, (nieder- oder mittelmolekulare) Polyisobutene (PIB), Polybutadiene, Isobuten-Isopren Copolymere (Butyl Rubber), Polyvinylethylether (PVE), Polyvinylbutylether, Copolymere von Vinylestern und Vinylethern, Styrol-Butadien-Copolymere (Styrol-Butadien-Rubber, SBR) oder Vinyl-Elastomere, z.B. auf Basis Vinylacetat/Vinyllaurat, Vinylacetat/Vinylstaerat oder Ethylen/Vinylacetat, sowie Mischungen der genannten Elastomere, wie beispielsweise in EP 0 242 325, US 4,518,615, US 5,093,136, US 5,266,336, US 5,601,858 oder US 6,986,709 beschrieben. Daneben umfassen erfindungsgemäß bevorzugt einzusetzende Kaugummibasen vorzugsweise weitere Bestandteile wie beispielsweise (mineralische) Füllstoffe, Weichmacher, Emulgatoren, Antioxidantien, Wachse, Fette oder fette Öle, wie beispielsweise gehärtete (hydrierte) pflanzliche oder tierische Fette, Mono-, Di- oder Triglyceride. Geeignete (mineralische) Füllstoffe sind beispielsweise Calciumcarbonat, Titandioxid, Siliciumdioxid, Talkum, Aluminiumoxid, Dicalciumphosphat, Tricalciumphosphat, Magnesiumhydroxid und deren Mischungen. Geeignete Weichmacher bzw. Mittel zur Verhinderung des Verklebens (detackifier) sind beispielsweise Lanolin, Stearinsäure, Natriumstearat, Ethylacetat, Diacetin (Gylcerindiacetat), Triacetin (Gylcerintriacetat), Triethylcitrat. Geeignete Wachse sind beispielsweise Paraffin Wachse, Candelilla Wachs, Carnauba Wachs, mikrokristalline Wachse und Polyethylen Wachse. Geeignete Emulgatoren sind beispielsweise Phosphatide wie Lecithin, Mono- und Diglyceride von Fettsäuren, z.B. Glycerinmonostearat.

Erfindungsgemäße Kaugummis (insbesondere wie vorstehend offenbart) umfassen vorzugsweise Bestandteile wie Zucker verschiedener Arten, Zuckeraustauschstoffe, andere süß schmeckende Stoffe, Zuckeralkohole (insbesondere Sorbitol, Xylitol, Mannitol), Kühlwirkstoffe, Geschmackskorrigenzien für unangenehme Geschmackseindrücke, weitere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropion-säure), Feuchthaltemittel, Verdicker, Emulgatoren, Stabilisatoren, Geruchskorrigentien und Aromen (z.B.: Eycalyptus-Menthol, Kirsche, Erdbeere, Grapefruit, Vanille, Banane, Citrus, Pfirsich, schwarze Johannisbeere, Tropenfrüchte, Ingwer, Kaffee, Zimt, Kombinationen (der genannten Aromen) mit Mintaromen sowie Spearmint und Pfefferminz alleine). Besonders interessant ist unter anderem die Kombination der Aromen mit weiteren Stoffen, die kühlende, wärmende und/oder mundwässerndernde Eigenschaften aufweisen.

Besonders bevorzugt ist ein erfindungsgemäßes oral konsumierbares Produkt (insbesondere Lebensmittel oder Futtermittel), wobei das oral konsumierbare Produkt ein Getränk ist,

wobei das Getränk vorzugsweise einen Zuckergehalt von 30 g/100 mL Getränk oder weniger, bevorzugt von 15 g/100 mL oder weniger, besonders bevorzugt 5 g/100 mL oder weniger aufweist, besonders bevorzugt kein Zucker enthält
und/oder
wobei das Getränk kein Ethanol oder maximal 0,1 Volumenprozent Ethanol enthält, bezogen auf das Volumen des Getränkes.

Im Rahmen der vorliegenden Erfindung sind erfindungsgemäße oral konsumierbare Produkte weniger bevorzugt, bei denen es sich um Ethanol enthaltende Getränke handelt.

Kein Ethanol bedeutet in der vorliegenden Erfindung, dass kein Ethanol zugesetzt wird und dass die Zubereitung weniger als 0,1 Vol.%, bevorzugt weniger als 0,01 Vol.-% und besonders bevorzugt keine messbare Menge an Ethanol enthält.

Besonders bevorzugt sind erfindungsgemäße oral konsumierbare Produkte (vorzugsweise Lebensmittel oder Futtermittel), wobei es sich um ein kohlensäurehaltiges Getränk oder um ein kohlensäurefreies Getränk handelt.

Eigene Untersuchungen haben gezeigt, dass der scharfe Geschmack und auch die Wirkung des N-Nonanoylvanillylamin auf den Schärferezeptor TRPV1 für die erfindungsgemäßen Wirkungen (Appetitreduzierung; Sättigung) nicht notwendig ist. Daher ist in bevorzugten oral konsumierbaren Produkten (insbesondere Lebensmitteln oder Futtermitteln) das N-Nonanoylvanillylamin mit einem weiteren Aromastoff, Lebensmittel,- Futtermittel-, oder Arzneimittelbestandteil kombiniert, der die TRPV1-Antwort reduziert oder vollständig aufhebt, , d.h. mit einem sogenannten TRPV1-Inhibitor, bevorzugt einem als Aromastoff zugelassenen TRPV1-Inhibitor.

Aromastoffe, Lebensmittel-, Futtermittel-, oder Arzneimittelbestandteile, die als TRPV1-Inhibitoren wirken, können z.B. sein: para-tert-Butylcyclohexanol nach WO 2009 087,242, Eriodictyol nach Rossato, M. F.; Trevisan, G.; Walker, C. I. B.; Klafke, J. Z.; de Oliveira, A. P.; Villarinho, J. G.; Zanon, R. B.; Royes, L. F. F.; Athayde, M. L.; Gomez, M. V.; Ferreira, J., Eriodictyol: A flavonoid antagonist of the TRPV1 receptor with antioxidant activity. Biochemical Pharmacotogy 2011, 814. (544-551) oder Eriodictyol enthaltende Pflanzenprodukte wie z.B. ein Extrakt aus Eriodictyon ssp. oder Citrus ssp.

Für die Dopaminfreisetzung durch N-Nonanoylvanillylamin ist das exemplarisch im nachfolgenden Beispiel 2 dargestellt; daraus geht hervor, dass die Dopaminfreisetzung nicht von der Gegenwart eines TRPV1-Inhibitors, in diesem Fall para-tert-Butylcyclohexanol, abhängig ist.

Die Erfindung betrifft auch eine erfindungsgemäße Stoffmischung oder ein erfindungsgemäßes oral konsumierbares Produkt (insbesondere Lebensmittel und Futtermittel), zusätzlich umfassend einen oder mehrere TRPV1-Inhibitoren, vorzugsweise ausgewählt aus der Gruppe bestehend aus trans-tert-Butylcyclohexanol und Eriodictyol,

In einer weiteren Ausgestaltung der vorliegenden Erfindung wird N-Nonanoylvanillylamin in Kombination mit zumindest einer Substanz zum Maskieren oder Vermindern eines unangenehmen (bitteren, metallischen, kalkigen, sauren, adstringierenden, scharfen) Geschmackseindrucks oder zur Verstärkung oder Erzeugung eines angenehmen Geschmackseindrucks (süß, salzig, Umami) verwendet.

Auf diese Weise kann eine Verstärkung des Geschmacks erreicht werden. Diese weiteren Substanzen können aus der folgenden Liste ausgewählt werden, ohne die Erfindung damit einzuschränken: trans-tert-Butylcyclohexanol nach WO 2009 087,242, Mononatriumglutamat, Glutaminsäure, Nucleotide (z.B. Adenosin-5'-monophosphat, Cytidin-5'-monophosphat, Inosin-5'-monophosphat, Guanosin-5'-monophosphat) oder deren pharmazeutisch akzeptable Salze, Lactisole, Hydroxyflavanone (z.B. Eriodictyol, Homoeriodictyol oder deren Natriumsalze), insbesondere gemäß EP 1 258 200, Hydroxybenzoesäureamide (z. B. 2,4-Dihydroxybenzoesäurevanillylamid, 4-Hydroxybenzoesäurevanillylamid), Gemische von Molkeproteinen mit Lecithinen, Hefeextrakte, Pflanzenhydrolysate, pulverisierte Gemüse (z.B. Zwiebelpulver, Tomatenpulver), Pflanzenextrakte (z.B. von Liebstöckel oder von Pilzen wie Shiitake), Meeralgen und Mineralsalzmischungen sowie Mischungen nach WO 2007/045,566.

In einer bevorzugten Ausgestaltung der vorliegenden Erfindung wird N-Nonanoylvanillylamin in den erfindungsgemäßen kalorienreduzierten Zusammensetzungen, Zubereitungen und Halbfertigwaren in Kombination mit zumindest einem süßverstärkenden Stoff eingesetzt, insbesondere mit einer oder mehreren Verbindungen gemäß WO 2007/014879 A1 oder WO 2007/107596 A1, speziell zusammen mit Hesperetin und/oder Phloretin. Hierdurch wird eine Verstärkung und eine Vertiefung sowie Abrundung des Geschmacksprofils erzielt. Der Gesamtanteil an Hesperetin und/oder Phloretin in solchen Zusammensetzungen oder Zubereitungen liegt vorzugsweise im Bereich von 1 bis 400 ppm, bevorzugt im Bereich von 5 - 200 ppm, bezogen auf das Gesamtgewicht der Zusammensetzung oder Zubereitung.

Zusätzlich zu einem oder mehreren süßverstärkenden Stoffen können in den erfindungsgemäßen Zusammensetzungen, Zubereitungen und Halbfertigwaren vorzugsweise Geschmackstoffe enthalten sein, die einen kribbelnden (tingling) oder kühlenden Effekt bewirken. So wurde bei der Kombination von N-Nonanoylvanillylamin mit Hesperetin und/oder Phloretin einerseits und cis- und/oder trans-Pellitorin (siehe WO 2004/000787 bzw. WO 2004/043906) andererseits ein weiter verbessertes und von Konsumenten bevorzugtes Geschmacksprofil erreicht. Der Gesamtanteil an cis- und/oder trans-Pellitorin in solchen Zusammensetzungen oder Zubereitungen liegt vorzugsweise im Bereich von 0,5 bis 500 ppm, bevorzugt im Bereich von 5 - 100 ppm, bezogen auf das Gesamtgewicht der Zusammensetzung oder Zubereitung.

Modulierende Aroma- und/oder Geschmackstoffe werden vorzugsweise ausgewählt aus der Gruppe bestehend aus trans-tert-Butylcyclohexanol, Adenosin-5'-monophosphat, Cytidin-5'-monophosphat, Inosin-5'-monophosphat, und deren pharmazeutisch akzeptablen Salzen; Lactisolen; 2,4-Dihydroxybenzoesäure; 3-Hydroxybenzoesäure; Natriumsalzen, vorzugsweise Natriumchlorid, Natriumlactat, Natriumcitrat, Natriumacetat, Natriumgluconoat; Hydroxyflavanonen, wie zum Beispiel Eriodictyol, Homoeriodictyol, und deren Natriumsalzen; Hydroxybenzoe-säureamiden, wie zum Beispiel 2,4-Dihydroxybenzoesäurevanillylamid, 2,4-Dihydroxybenzoesäure-N-(4-hydroxy-3-methoxybenzyl)amid, 2,4,6-Trihydroxybenzoesäure-N-(4-hydroxy-3-methoxybenzyl)amid, 2-Hydroxy-benzoesäure-N-4-(hydroxy-3-methoxybenzyl)amid, 4-Hydroxybenzoesäure-N-(4-hydroxy-3-methoxybenzyl)-amid, 2,4-Dihydroxybenzoesäure-N-(4-hydroxy-3-methoxybenzyl)amid-Mono-natriumsalz, 2,4-Dihydroxybenzoesäure-N-2-(4-hydroxy-3-methoxyphenyl)-ethyl-amid, 2,4-Dihydroxybenzoesäure-N-(4-hydroxy-3-ethoxybenzyl)amid, 2,4-Dihydroxybenzoesäure-N-(3,4-dihydroxybenzyl)amid und 2-Hydroxy-5-methoxy-N-[2-(4-hydroxy-3-methoxyphenyl)ethyl]amid; 4-Hydroxy-benzoesäurevanillylamid (insbesondere solche wie beschrieben in WO 2006/024587, die bezüglich der darin offenbarten entsprechenden Verbindungen auf dem Wege der Verweisung Bestandteil dieser Anmeldung wird); Hydroxydeoxybenzoinen, wie zum Beispiel 2-(4-Hydroxy-3-methoxyphenyl)-1-(2.4,6-trihydroxyphenyl)ethanon, 1-(2,4-Dihydroxyphenyl)-2-(4-hydroxy-3-methoxy-phenyl)ethanon, 1-(2-Hydroxy-4-methoxyphenyl)-2-(4-hydroxy-3-methoxyphenyl) ethanon) (insbesondere solche wie beschrieben in WO 2006/106023, die bezüglich der darin offenbarten entsprechenden Verbindungen auf dem Wege der Verweisung Bestandteil dieser Anmeldung wird); Hydroxyphenlaalkandionen, wie zum Beispiel Gingerdion-[2], Gingerdion-[3], Gingerdion-[4], Dehydrogingerdion-[2], Dehydrogingerdion-[3], Dehydrogingerdion-[4]) (insbesondere solche wie beschrieben in WO 2007/003527, die bezüglich der darin offenbarten entsprechenden Verbindungen auf dem Wege der Verweisung Bestandteil dieser Anmeldung wird); Diacetyltrimeren (insbesondere solche wie beschrieben in WO 2006/058893, die bezüglich der darin offenbarten entsprechenden Verbindungen auf dem Wege der Verweisung Bestandteil dieser Anmeldung wird); gamma-Aminobuttersäuren (insbesondere solche wie beschrieben in WO 2005/096841, die bezüglich der darin offenbarten entsprechenden Verbindungen auf dem Wege der Verweisung Bestandteil dieser Anmeldung wird) und Divanillinen (insbesondere Divanillin wie beschrieben in WO 2004/078302, die bezüglich der darin offenbarten entsprechenden Verbindungen auf dem Wege der Verweisung Bestandteil dieser Anmeldung wird); Bicyclo[4.1.0]heptan-7-carbonsäureamide, insbesondere solche wie beschrieben in EP 2 079 322 (Symrise), die bezüglich der darin offenbarten entsprechenden Ver-bindungen auf dem Wege der Verweisung Bestandteil dieser Anmeldung wird; Cyclopropancarbonsäure(3-methyl-cyclohexyl)amide, insbesondere solche wie beschrieben in EP 1 989 944 (Symrise), die bezüglich der darin offenbarten entsprechenden Verbindungen auf dem Wege der Verweisung Bestandteil dieser Anmeldung wird; Aromatische Neo-Menthylamide, insbesondere solche wie beschrieben in EP 2 064 959 (Symrise), die bezüglich der darin offenbarten entsprechenden Verbindungen auf dem Wege der Verweisung Bestandteil dieser Anmeldung wird; Neo-Menthylamide, insbesondere solche wie beschrieben in US 2009/311401 A (Symrise), die bezüglich der darin offenbarten entsprechenden Verbindungen auf dem Wege der Verweisung Bestandteil dieser Anmeldung wird.

Weitere Aspekte der vorliegenden Erfindung ergeben sich aus den nachfolgenden Beispiele und den beigefügten Ansprüchen.

### Figurenbeschreibung

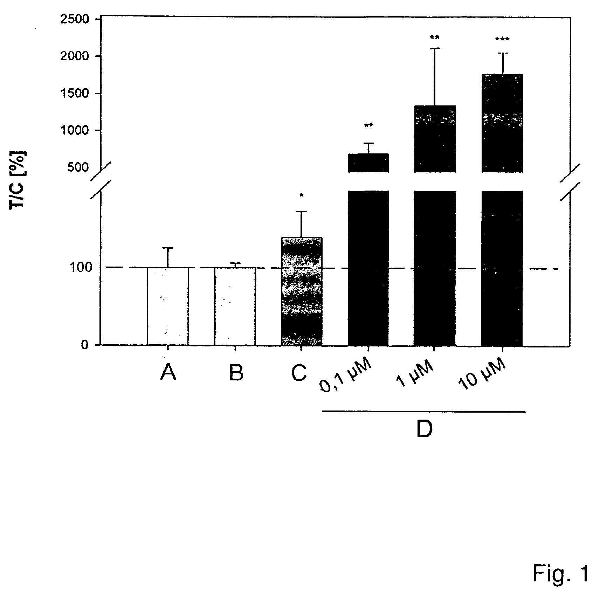

Fig. 1 stellt die Dopamin-Freisetzung durch SH-SY5Y-Zellen nach Stimulierung mit 100 µM Acetylcholin (Positivkontrolle) oder verschiedenen Konzentrationen von N-Nonanoylvanillylamin (Nonivamid) dar. Die Ergebnisse werden normalisiert auf die Kontrolle in % dargestellt (T/C [%]). n= 6 (3 biologische und 2 technische Replikate); * p ≤ 0,05 vs. Kontrolle ** p ≤ 0,01 vs. Kontrolle *** p ≤ 0,001 vs. Kontrolle. Die in der Figur verwendeten Buchstaben bedeuten: A= Kontrolle (Puffer), B= EtOH Kontrolle (Puffer 0,1 % EtOH), C= Acetylcholin 100µM, D = N-Nonanoylvanillylamin 0,1 µM, 1 µM, bzw. 10 µM. Die tabellarische Form der Daten zu Fig. 1 ist nachfolgend angegeben:

| Testsubstanz | T/C [%] | Standardabweichung |
|---|---|---|
| Kontrolle | 100 | 25,72 |
| Ethanolkontrolle | 100 | 5,99 |
| Acetylcholin 100 µM (Positivkontrolle) | 139,84 | 32,91 |
| N-Nonanoylvanillylamin 0,1 µM | 697,84 | 145,14 |
| N-Nonanoylvanillylamin 1 µM | 1350,94 | 775,88 |
| N-Nonanoylvanillylamin 10 µM | 1774,98 | 290,45 |

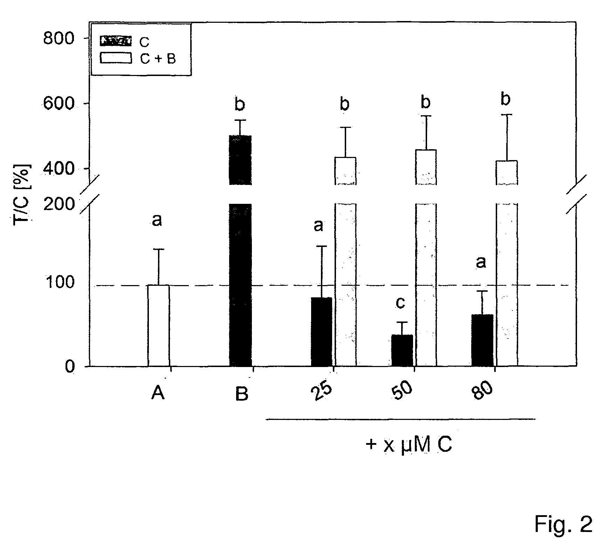

Fig. 2 stellt die Dopamin-Freisetzung durch SH-SY5Y-Zellen nach Stimulierung mit 1 µM N-Nonanoylvanillylamin (Nonivamid) beziehungsweise mit 1 µM N-Nonanoylvanillylamin (Nonivamid) und verschiedenen Konzentrationen des selektiven TRPV1-Inhibitors trans-4-tert-Butylcyclohexanol dar. Die Ergebnisse werden normalisiert auf die Kontrolle in % dargestellt (T/C [%]). n= 4 (2 biologische und 2 technische Replikate). Signifikante Unterschiede zwischen den Behandlungen wurden mittels Student's T-Test ermittelt und werden durch die Buchstaben a,b und c dargestellt. Die in der Figur verwendeten Buchstaben bedeuten:, A = Kontrolle (Puffer), B = N-Nonanoylvanillylamin 1 µM, C = 25 µM trans-4-tert-Butylcyclohexanol, 50µM trans-4-tert-Butylcyclohexanol, 80 µM trans-4-tert-Butylcyclohexanol, C + B = trans-4-tert-Butylcyclohexanol + 1µM N-Nonanoylvanillylamin.
Die tabellarische Form der Daten zu Fig. 2 ist nachfolgend angegeben:

| Testsubstanz | T/C [%] | Standardabweichung |
|---|---|---|
| Kontrolle | 100,0 | 44,44 |
| N-Nonanoylvanillylamin 1 µM | 501,20 | 47,86 |
| N-Nonanoylvanillylamin 1 µM + 25 µM *trans*-tert-Butylcyclohexanol | 433,55 | 92,86 |
| N-Nonanoylvanillylamin 1 µM + 50 µM *trans*-4-tert-Butylcyclohexanol | 456,80 | 105,52 |
| N-Nonanoylvanillylamin 1 µM + 80 µM *trans*-4-tert-Butylcyclohexanol | 422,48 | 143,41 |
| 25 µM *trans*-4-tert-Butylcyclohexanol | 84,47 | 63,77 |
| 50 µM *trans*-4-tert-Butylcyclohexanol | 38,19 | 16,06 |
| 80 µM *trans*-4-tert-Butylcyclohexanol | 63,53 | 29,42 |

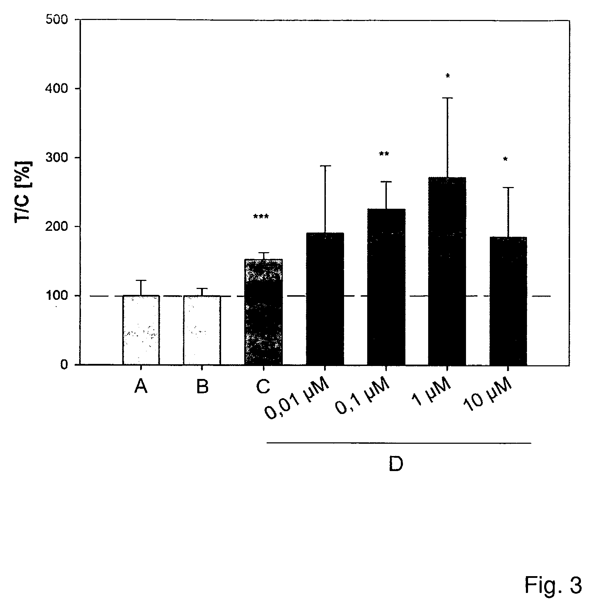

Fig. 3 stellt die Serotoninfreisetzung durch SH-SY5Y-Zellen nach Stimulierung mit 50 mM Kaliumchlorid (50 mM KCl, Positivkontrolle) oder verschiedenen Konzentrationen von N-Nonanoylvanillylamin dar. Die Ergebnisse werden normalisiert auf die Kontrolle in % dargestellt (T/C [%]). n= 6 (3 biologische und 2 technische Replikate); * p ≤ 0,05 vs. Kontrolle ** p ≤ 0,01 vs. Kontrolle *** p ≤ 0,001 vs. Kontrolle. Die in der Figur verwendeten Buchstaben bedeuten: A= Kontrolle (Puffer), B= EtOH Kontrolle (Puffer 0,1 % EtOH), C= 50 nM KCl, D = N-Nonanoylvanillylamin 0,01 µM, 0,1 µM, 1 µM, bzw. 10 µM.
Die tabellarische Form der Daten zu Fig. 3 ist nachfolgend wiedergegeben:

| Testsubstanz | T/C [%] | Standardabweichung |
|---|---|---|
| Kontrolle | 100,00 | 22,31 |
| Ethanolkontrolle | 100,00 | 10,80 |
| 50 mM KCl (Positivkontrolle) | 153,48 | 9,9 |
| N-Nonanoylvanillylamin 0,01 µM | 191,35 | 98,18 |
| N-Nonanoylvanillylamin 0,1 µM | 226,44 | 39,71 |
| N-Nonanoylvanillylamin 1 µM | 272,12 | 115,53 |
| N-Nonanoylvanillylamin 10 µM | 185,65 | 72,76 |

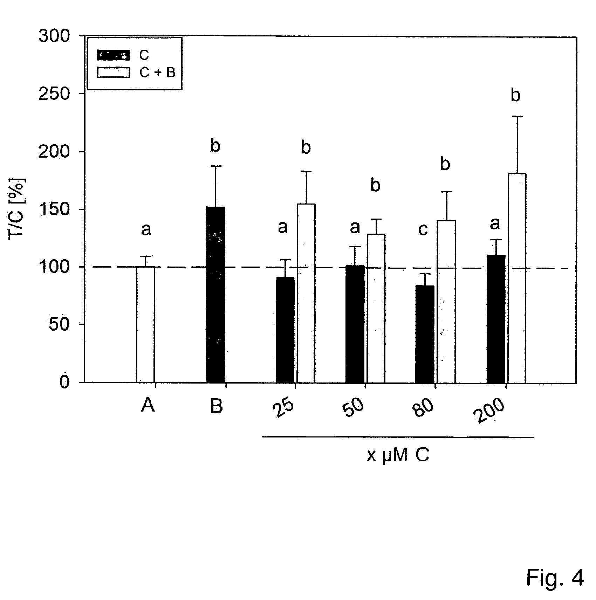

Fig. 4 stellt die Serotoninfreisetzung durch SH-SY5Y-Zellen nach Stimulierung mit 1 µM N-Nonanoylvanillylamin (Nonivamid) beziehungsweise mit 1 µM N-Nonanoylvanillylamin (Nonivamid) und verschiedenen Konzentrationen des selektiven TRPV1-Inhibitors trans-4-tert-Butylcyclohexanol dar. Ergebnisse werden normalisiert auf die Kontrolle in % dargestellt (T/C [%]). n= 4 (2 biologische und 2 technische Replikate). Signifikante Unterschiede zwischen den Behandlungen wurden mittels Student's T-Test ermittelt und werden durch die Buchstaben a, b und c dargestellt. Die in der Figur verwendeten Buchstaben bedeuten: A = Kontrolle (Puffer), B = N-Nonanoylvanillylamin 1 µM, C = 25 µM 50µM 80 µM trans-4-tert-Butylcyclohexanol, bzw. 200 µM trans-4-tert-Butylcyclohexanol, C + B = trans-4-tert-Butylcyclohexanol + 1µM N-Nonanoylvanillylamin.
Die tabellarische Form der Daten zu Fig. 4 ist nachfolgend angegeben:

| Testsubstanz | T/C [%] | Standardabweichung |
|---|---|---|
| Kontrolle | 100,00 | 8,97 |
| N-Nonanoylvanillylamin 1 µM | 151, 75 | 35,46 |
| N-Nonanoylvanillylamin 1 µM + 25 µM *trans*-tert-Butylcyclohexanol | 154,86 | 28,13 |
| N-Nonanoylvanillylamin 1 µM + 50 µM *trans*-tert-Butylcyclohexanol | 128, 74 | 13,12 |
| N-Nonanoylvanillylamin 1 µM + 80 µM *trans*-tert-Butylcyclohexanol | 140,81 | 24,92 |
| N-Nonanoylvanillylamin 1 µM + 200 µM *trans*-tert-Butylcyclohexanol | 181,93 | 49,64 |
| 25 µM *trans*-tert-Butylcyclohexanol | 91,07 | 15,20 |
| 50 µM *trans*-tert-Butylcyclohexanol | 101,39 | 16,59 |
| 80 µM *trans*-tert-Butylcyclohexanol | 84,129 | 10,73 |
| 200 µM *trans*-tert-Butylcyclohexanol | 110,99 | 13,76 |

### Beispiele

Als Zellmodell werden humane Neuroblastomazellen (SH-SY5Y, ATCC Nummer CRL-2266) verwendet. Die Kultivierung erfolgt bei 37°C und 5 % CO₂-Gehalt mit einer Mischung, die zu gleichen Teilen aus Eagle's Minimum Essential Medium (MEM) und F12 Medium (jeweils mit 10 % FBS und 1 % Penicillin / Streptomycin) besteht. Für die Messung der Dopamin-und Serotoninfreisetzung werden die Zellen mit Trypsin geerntet und nach einer Vitalitätsprüfung durch Trypanblaufärbung in definierter Zellzahl in 35 mm Zellkulturschalen ausgesät.

### Beispiel 1: Freisetzung von Dopamin in einem experimentellen Zellsystem mittels N-Nonanoylvanillylamin

Methode zur Messung der Freisetzung von Dopamin:
Nach 3-minütiger Stimulierung von 1,25 * 10⁶ humanen Neuroblastomazellen (SH-SY5Y) mit 350µL Krebs-Ringer-HEPES Puffer, pH 5, mit oder ohne Zusatz von N-Nonanoylvanillylamin (wobei bei Zusatz von N-Nonanoylvanillylamin Konzentrationen von 0,1µM, 1µM und 10µM im Krebs-Ringer-HEPES Puffer eingestellt werden) wird der Überstand mit 1 N HCl angesäuert und der Dopamingehalt mit Hilfe eines enzymbasierten Nachweisverfahrens (Dopamin-ELISA, DLD Diagnostica, Hamburg, Deutschland) ermittelt. Die Zellen werden mit einem natriumlaurylsarcosinathaltigen Puffer lysiert und der DNA-Gehalt mit Hilfe einer NanoQuant-Platte (Tecan, Mennendorf, Schweiz) zur Normalisierung der Dopaminfreisetzung ermittelt. Bei einer Ethanolkontrolle (EtOH-Kontrolle) wird kein N-Nonanoylvanillylamin zum Krebs-Ringer-HEPES Puffer hinzugefügt, sondern der Puffer wird mit Ethanol versetzt, sodass eine 0,1% ethanolische Krebs-Ringer-HEPES Puffer-Lösung resultiert. Bei der Positivkontrolle wird anstatt des N-Nonanoylvanillylamins Acetylcholin verwendet, wobei eine Konzentration von 100µM Acetylcholin im Krebs-Ringer-HEPES Puffer eingestellt wird.

**Tabelle 1. Daten zu Fig. 1 (Dopaminfreisetzung durch SH-SY5Y-Zellen nach Stimulierung mit 100 µM Acetylcholin (Positivkontrolle) oder verschiedenen Konzentrationen von N-Nonanoylvanillylamin (Nonivamid)).**

| Testsubstanz | T/C [%] | Standardabweichung |
|---|---|---|
| Kontrolle | 100 | 25,72 |
| EtOH Kontrolle | 100 | 5,99 |
| Acetylcholin 100 µM | 139,84 | 32,91 |
| N-Nonanoylvanillylamin 0,1 µM | 697,84 | 145,14 |
| N-Nonanoylvanillylamin 1 µM | 1350,94 | 775,88 |
| N-Nonanoylvanillylamin 10 µM | 1774,98 | 290,45 |

In Fig. 1 wird der sehr starke Einfluss geringer Konzentrationen von N-Nananoylvanillylamin auf die Dopaminfreisetzung deutlich. Bereits eine Konzentration von 100 nM des N-Nonanoylvanillylamin führt zu einer siebenfachen Steigerung der Dopaminfreisetzung durch SH-SY5Y-Zellen.

### Beispiel 2: Freisetzung von Dopamin in einem experimentellen Zellsystem mit N-Nonanoylvanillylamin und einem TRPV1-Inhibitor

Nach 3-minütiger Stimulierung von 1,25 * 10⁶ humanen Neuroblastomazellen (SH-SY5Y) mit 350 µL Krebs-Ringer-HEPES Puffer, pH 5, mit oder ohne Zusatz von 1 µM N-Nonanoylvanillylamin (wobei bei Zusatz von N-Nonanoylvanillylamin eine Konzentration von 1µM im Krebs-Ringer-HEPES Puffer eingestellt wird) in Kombination mit verschieden anwendungsrelevanten Mengen des selektiven TRPV1-Inhibitors trans-4-tert-Butylcyclohexanol (sodass Konzentrationen von 25, 50 und 80 µM des trans-4-tert-Butylcyclohexanols in dem Krebs-Ringer-HEPES Puffer eingestellt werden) wird der Überstand mit 1 N HCl angesäuert und der Dopamingehalt mit Hilfe eines enzymbasierten Nachweisverfahrens (Dopamin-ELISA, DLD Diagnostica, Hamburg, Deutschland) ermittelt. Die Zellen werden mit einem natriumlaurylsarcosinathaltigem Puffer lysiert und der DNA-Gehalt mit Hilfe einer NanoQuant-Platte (Tecan, Mennendorf, Schweiz) zur Normalisierung der Dopaminfreisetzung ermittelt. Zum Vergleich wurden die Versuche ohne die Zugabe von N-Nonanoylvanillylamin wiederholt.

**Tabelle 2. Dopamin-Freisetzung SH-SY5Y-Zellen nach Stimulierung mit 1 µM N-Nonanoylvanillylamin (Nonivamid) und verschiedenen Konzentrationen des selektiven TRPV1-Inhibitors trans-4-tert-Butylcyclohexanol**

| Testsubstanz | T/C [%] | Standardabweichung |
|---|---|---|
| Kontrolle | 100,0 | 44,44 |
| N-Nonanoylvanillylamin 1 µM | 501,20 | 47,86 |
| N-Nonanoylvanillylamin 1 µM + 25 µM trans-4-tert-Butylcyclohexanol | 433,55 | 92,86 |
| N-Nonanoylvanillylamin 1 µM + 50 µM trans-4-tert-Butylcyclohexanol | 456,80 | 105,52 |
| N-Nonanoylvanillylamin 1 µM + 80 µM trans-4-tert-Butylcyclohexanol | 422,48 | 143,41 |
| 25 µM trans-4-tert-Butylcyclohexanol | 84,47 | 63,77 |
| 50 µM trans-4-tert-Butylcyclohexanol | 38,19 | 16,06 |
| 80 µM trans-4-tert-Butylcyclohexanol | 63,53 | 29,42 |

Die fünffache Steigerung der Dopaminfreisetzung durch 1 µM N-Nonanoylvannillylamin wurde durch gleichzeitige Stimulierung mit dem selektiven TRPV1-Inhibitors trans-4-tert-Butylcyclohexanol in den anwendungsrelevanten Konzentrationen (25, 50 und 80 µM) nicht signifikant beeinflusst. Die Daten zeigen somit, dass der für den Scharfgeschmack verantwortliche TRPV1-Rezeptor nicht für die durch N-Nonanoylvanillylamin gesteigerte Dopaminausschüttung notwendig ist.

### Beispiel 3: Freisetzung von Serotonin in einem experimentellen Zellsystem mit N-Nonanoylvanillylamin

Nach 3-minütiger Stimulierung von 1,25 * 10⁶ humanen Neuroblastomazellen (SH-SY5Y) mit 300µL Krebs-Ringer-HEPES Puffer, 0,1 % Ascorbinsäure, pH 6,2, mit oder ohne Zusatz N-Nonanoylvanillylamin (wobei bei Zusatz von N-Nonanoylvanillylamin Konzentrationen von 0,01µM, 0,1 µM, 1µM und 10 µM im Krebs-Ringer-HEPES Puffer eingestellt werden) wird der Serotoningehalt mit Hilfe eines enzymbasierten Nachweisverfahrens (Serotonin-ELISA sensitiv, DLD Diagnostica, Hamburg, Deutschland) ermittelt. Die Zellen werden mit einem natriumlaurylsarcosinathaltigem Puffer lysiert und der DNA-Gehalt mit Hilfe einer NanoQuant-Platte (Tecan, Mennendorf, Schweiz) zur Normalisierung der Serotoninfreisetzung ermittelt.

**Tabelle 3. Serotonin-Freisetzung SH-SY5Y-Zellen nach Stimulierung mit 50 mM Kaliumchlorid (50 mM KCl, Positivkontrolle) oder verschiedenen Konzentrationen von N-Nonanoylvanillylamin (Nonivamid).**

| Testsubstanz | T/C [%] | Standardabweichung |
|---|---|---|
| Kontrolle | 100,00 | 22,31 |
| EtOH Kontrolle | 100,00 | 10,80 |
| 50 mM KCl | 153,48 | 9,9 |
| N-Nonanoylvanillylamin 0,01 µM | 191,35 | 98,18 |
| N-Nonanoylvanillylamin 0,1 µM | 226,44 | 39,71 |
| N-Nonanoylvanillylamin 1 µM | 272,12 | 115,53 |
| N-Nonanoylvanillylamin 10 µM | 185,65 | 72,76 |

Ab einer Konzentration von 0,1 µM N-Nonanoylvanillylamin steigt die Serotoninausschüttung durch SH-SY5Y Zellen hoch signifikant an, d.h. p < 0,005.

### Beispiel 4: Freisetzung von Serotonin in einem experimentellen Zellsystem mit N-Nonanoylvanillylamin und einem TRPV1-Inhibitor

Nach 3-minütiger Stimulierung von 1,25 * 10⁶ humanen Neuroblastoma Zellen (SH-SY5Y) mit 300 µL Krebs-Ringer-HEPES Puffer (0,1 % Ascorbinsäure, pH 6,2) mit oder ohne Zusatz von N-Nonanoylvanillylamin (sodass eine Konzentration von 1 µM N-Nonanoylvanillylamin im Krebs-Ringer-HEPES Puffer eingestellt wird) in Kombination mit verschieden anwendungsrelevanten Konzentrationen des selektiven TRPV1-Inhibitors trans-4-tert-Butylcyclohexanol wird der Serotoningehalt mit Hilfe eines enzymbasierten Nachweisverfahrens (Serotonin-ELISA sensitiv, DLD Diagnostica, Hamburg, Deutschland) ermittelt. Die Zellen werden mit einem natriumlaurylsarcosinathaltigem Puffer lysiert und der DNA-Gehalt mittels der NanoQuant-Platte in einem Plattenlesegerät (Tecan, Mennendorf, Schweiz) zur Normalisierung der Serotoninfreisetzung ermittelt.

**Tabelle 4. Daten zu Serotoninfreisetzung durch SH-SY5Y-Zellen nach Stimulierung mit 1 µM N-Nonanoylvanillylamin oder verschiedenen Konzentrationen von N-Nonanoylvanillylamin (Nonivamid).**

| Testsubstanz | T/C [%] | Standardabweichung |
|---|---|---|
| Kontrolle | 100,00 | 8,97 |
| N-Nonanoylvanillylamin 1 µM | 151,75 | 35,46 |
| N-Nonanoylvanillylamin 1 µM + 25 µM trans-4-tert-Butylcyclohexanol | 154,86 | 28,13 |
| N-Nonanoylvanillylamin 1 µM | 128,74 | 13,12 |
| + 50 µM trans-4-tert-Butylcyclohexanol | | |
| N-Nonanoylvanillylamin 1 µM | 140,81 | 24,92 |
| + 80 µM trans-4-tert-Butylcyclohexanol | | |
| N-Nonanoylvanillylamin 1 µM | 181,93 | 49,64 |
| + 200 µM trans-4-tert-Butylcyclohexanol | | |
| 25 µM trans-4-tert-Butylcyclohexanol | 91,07 | 15,20 |
| 50 µM trans-4-tert-Butylcyclohexanol | 101,39 | 16,59 |
| 80 µM trans-4-tert-Butylcyclohexanol | 84,129 | 10,73 |
| 200 µM trans-4-tert-Butylcyclohexanol | 110,99 | 13,76 |

Die vorliegenden Daten zeigen, dass die Serotoninausschüttung in SH-SY5Y-Zellen durch N-Nonanoylvanillyamin durch Zugabe des selektiven TRPV1-Inhibitors trans-4-tert-Butylcyclohexanol nicht signifikant beeinflusst wird. Der für den Scharfgeschmack verantwortliche TRPV1-Rezeptor ist somit an der durch N-Nonanoylvanillyamin verursachten Serotoninausschüttung in SH-SY5Y Zellen nicht beteiligt.

### Anwendunasbeispiele:

### Anwenduncisbeispiel 1: Erfrischungsgetränke (zuckerhaltig, kalorienreduziert, kalorienfrei)

| Inhaltsstoff | Einsatz in Gew.-% | | | | | | |
|---|---|---|---|---|---|---|---|
| Zubereitung | A | B | C | D | E | F | G |
| Zucker (Sucrose) | 10 | 10 | 7 | - | - | 8 | 7 |
| Glucose/Fructose-Sirup aus Mais, enthaltend 55 Gew.-% Fructose | - | - | - | - | 10 | - | - |
| Rebaudiosid A 95 % | - | - | 0,02 | 0,05 | - | - | - |
| Zitronensäure | 0,15 | 0,15 | 0,06 | 0,15 | 0,15 | 0,15 | 0,15 |
| Phosphorsäure | - | - | 0,07 | - | - | - | - |
| Zuckercouleur | - | - | 0,14 | - | - | - | - |
| Coffein | - | - | 0,01 | - | - | - | - |
| Zitronenaroma | 0,1 | 0,05 | - | 0,1 | 0,1 | 0,1 | 0,1 |
| Limonenaroma | - | 0,05 | - | - | - | - | - |
| Getränke-Emulsion Typ "Cola" | - | - | 0,05 | - | - | - | - |
| Phloretin | - | - | 0,002 | 0,003 | - | 0,002 | 0,001 |
| Hesperetin | - | - | 0,001 | 0,002 | - | - | 0,002 |
| Extrakt aus Rubus suavissimus, enthaltend 5 Gew.-% Rubusosid bez. auf das Gesamtgewicht des Extraktes | - | - | - | - | - | 0,01 | - |
| Homoeriodictyol-Natriumsalz | - | - | 0,005 | 0,005 | - | - | - |
| N-Nonanoylvanillylamin | 0,000003 | 0,0001 | 0,00005 | 0,00001 | 0,000003 | 0,0001 | 0,00005 |
| Eriodictyol | - | 0,0100 | 0,0100 | - | - | 0,0100 | 0,0100 |
| Wasser | auf 100 auffüllen | | | | | | |

Die Zutaten wurden in der angegebenen Reihenfolge gemischt und mit Wasser auf 100 % aufgefüllt. Die Mischungen werden in Glasflaschen gefüllt und carbonisiert.

**Anwendungsbeispiel 2: Verwendung in einem Kaugummis**

| | | Einsatz in Gew.-% | |
|---|---|---|---|
| Teil | Inhaltsstoff | A | B |
| A | Kaugummibase, Company "Jagum T" | 30,4899 | 30,49999 |
| B | Sorbit, pulverisiert | 39,00 | 39,00 |
| | Isomalt® (Palatinit GmbH) | 9,50 | 9,50 |
| | Xylit | 2,00 | 2,00 |
| | Mannit | 3,00 | 3,00 |
| | Aspartam® | 0,10 | 0,10 |
| | Acesulfam® K | 0,10 | 0,10 |
| | Emulgum® (Colloides Naturels, Inc.) | 0,30 | 0,30 |
| C | Sorbitol, 70% | 14,00 | 14,00 |
| | Glycerin | 1,00 | 1,00 |
| D | Pfefferminzaroma | 0,5 | 0,5 |
| | N-Nonanoylvanillylamin | 0,0001 | 0,00001 |
| | Eriodictyol | 0,0100 | - |

Teile A bis D werden gemischt und intensiv geknetet. Die Rohmasse kann z.B. in Form von dünnen Streifen zu verzehrsfertigen Kaugummis verarbeitet werden.

**Anwendungsbeispiel 3: Verwendung in Hartkaramellen**

| Inhaltsstoff | Gehalt (Gew.-%) | | | |
|---|---|---|---|---|
| | A | B | C | D |
| Zucker | 74,50 | - | - | - |
| Palatinit, Typ M | - | 74,00 | 75,50 | 75,00 |
| Zitronensäure | 0,5 | 1,0 | 0,5 | - |
| Farbstoff gelb | - | 0,01 | - | - |
| Farbstoff rot | - | - | 0,01 | - |
| Farbstoff blau | 0,01 | - | - | 0,01 |
| Pfefferminzaroma | 0,1 | - | - | 0,1 |
| Zitronenaroma | - | 0,1 | - | - |
| Rotfruchtaroma | - | - | 0,1 | - |
| Rebaudiosid A 98 % | - | 0,040 | - | 0,040 |
| Balansin A nach [SY317] | - | 0,005 | 0,010 | 0,005 |
| Hesperetin | - | 0,001 | - | 0,001 |
| Phloretin | - | 0,002 | - | - |
| N-Nonanoylvanillylamin | 0,0001 | 0,00001 | 0,000005 | 0,0001 |
| Eriodictyol | 0,0100 | - | - | - |
| para-4-tert-Butylcyclohexanol | - | - | - | 0,0100 |
| Wasser | ad 100 | ad 100 | ad 100 | ad 100 |

Palatinit bzw. der Zucker wurden ggfs. nach Zugabe der Zitronensäure mit Wasser gemischt und die Mischung bei 165 °C aufgeschmolzen und anschließend auf 115°C abgekühlt. Das Aroma und die anderen Bestandteile wurden zugegeben und nach dem Durchmischen in Formen gegossen, nach dem Erstarren aus den Formen entfernt und anschließend einzeln verpackt.

**Anwendungsbeispiel 4: Fettarme Joghurts**

| | Zubereitung (Angaben als Gew.-%) | | | |
|---|---|---|---|---|
| Inhaltsstoff | A | B | C | D |
| Sucrose | 10 | 8 | 6 | - |
| Rebaudiosid A 98 % | - | - | - | 0,050 |
| Extrakt aus Rubus suavissimus, enthaltend 5 Gew.-% Rubusosid bezogen auf das Gesamtgewicht des Extraktes, z.B. von Plant Extrakt | - | 0,010 | 0,010 | - |
| Hesperetin | - | 0,001 | 0,001 | 0,002 |
| Phloretin | - | - | 0,002 | 0,002 |
| Homoeriodictyol-Natriumsalz | - | - | - | 0,005 |
| N-Nonanoylvanillylamin | 0,00001 | 0,0001 | 0,000005 | 0,0001 |
| Eriodictyol | | 0,0100 | | 0,0100 |
| Joghurt, 0,1 % Fett | zu 100 % auffüllen | | | |

Die Inhaltsstoffe wurden gemischt und auf 5°C gekühlt.

**Anwendungsbeispiel 5: Fruchtgummis**

| | Zubereitung (Angaben als Gew.-%) | |
|---|---|---|
| Inhaltsstoff | A | B |
| Saccharose | 34,50 | 8,20 |
| Glucosesirup, DE 40 | 31,89 | 30,09 |
| Iso Syrup C* Tru Sweet 01750 (Cerestar GmbH) | 1,50 | 2,10 |
| Gelatine 240 Bloom | 8,20 | 9,40 |
| Polydextrose (Litesse® Ultra, Danisco Cultor GmbH) | - | 24,40 |
| Gelber und roter Farbstoff | 0,01 | 0,01 |
| Citronensäure | 0,20 | |
| Kirscharoma, enthaltend 1 Gew.-% Hesperetin 2 und 0,3 Gew.-% Phloretin bezogen auf das Aroma | - | 0,10 |
| N-Nonanoylvanillylamin | 0,0001 | 0,00001 |
| Eriodictyol | 0,0100 | |
| Wasser | ad 100 | ad 100 |

Polydextrose ist ein nicht süß schmeckendes Polysaccharid mit niedrigem Brennwert.

## Patentansprüche

1. Nicht-therapeutische Verwendung von N-Nonanoylvanillylamin enthalten in einem oral konsumierbaren Produkt, das kein Capsaicin enthält, in einer Konzentration von 0,05 - 0,001 mg/kg bezogen auf die Gesamtmasse des oral konsumierbaren Produktes zur Reduzierung des Appetits und zur Vermittlung eines Gefühls von Sättigung.

2. Verfahren zur nicht-therapeutischen Reduzierung des Appetits und zur nicht-therapeutischen Vermittlung eines Gefühls von Sättigung, bei dem man N-Nonanoylvanillylamin enthalten in einem oral konsumierbaren Produkt, das kein Capsaicin enthält, in einer Konzentration von 0,05 - 0,001 mg/kg bezogen auf die Gesamtmasse des oral konsumierbaren Produktes an ein menschliches oder tierisches Individuum abgibt.

3. Oral konsumierbares Produkt, umfassend N-Nonanoylvanillylamin und einen oder mehrere weitere Stoffe, wobei
(i) das N-Nonanoylvanillylamin in einer den Appetit reduzierenden und/oder ein Gefühl von Sättigung bewirkenden Konzentration bezogen auf die Gesamtmasse des oral konsumierbaren Produktes von 0,05 mg/kg oder weniger und gleichzeitig in einer Konzentration von zumindest 0,001 mg/kg vorliegt,
(ii) das oral konsumierbare Produkt kein Capsaicin umfasst,
(iii) und nicht mehr als 200 kcal/100g oral konsumierbaren Produkts enthält.

4. Oral konsumierbares Produkt nach Anspruch 3, **dadurch gekennzeichnet, dass** es außer N-Nonanoylvanillylamin keine weiteren Capsaicinoide enthält.

5. Oral konsumierbares Produkt nach mindestens einem der Ansprüche 3 bis 4, **dadurch gekennzeichnet, dass** es nicht mehr als 100 kcal/100g oral konsumierbaren Produkts enthält.

6. Oral konsumierbares Produkt nach mindestens einem der Ansprüche 3 bis 5, **dadurch gekennzeichnet, dass** es ausgewählt ist aus der Gruppe bestehend aus Süßwaren, nicht-alkoholischen Getränke, Instantgetränken, Getreideprodukten, Milchprodukten, Produkten aus Sojaprotein oder anderen Sojabohnen-Fraktionen, Süßstoffzubereitungen, Süßstofftabletten und Süßstoffsachets, Eiscremes und Dragees.

7. Oral konsumierbares Produkt nach mindestens einem der Ansprüche 3 bis 6, **dadurch gekennzeichnet, dass** das oral konsumierbare Produkt Joghurt, Kefir oder Quark darstellt.

8. Oral konsumierbares Produkt nach Anspruch 7, **dadurch gekennzeichnet, dass** es
(i) Früchte und/oder Fruchtzubereitungen und/oder
(ii) Zucker und/oder
(iii) Verdickungsmittel und/oder
(iv) Geliermittel und/oder
(v) Süßungsmittel und/oder
(vi) Aromen und/oder
(vii) Konservierungsstoffe
enthält.

9. Oral konsumierbares Produkt nach mindestens einem der Ansprüche 6 bis 8, **dadurch gekennzeichnet, dass** es ein Probiotikum enthält.

10. Oral konsumierbares Produkt nach mindestens einem der Ansprüche 3 bis 5, **dadurch gekennzeichnet, dass** es eine Kaugummibase enthält.

11. Oral konsumierbares Produkt nach mindestens einem der Ansprüche 3 bis 5, **dadurch gekennzeichnet, dass** es ein Getränk darstellt.

12. Zubereitungen nach Anspruch 3, **dadurch gekennzeichnet, dass** sie des Weiteren einen oder mehrere TRPV1-Inhibitoren enthalten.

## Claims

1. Non-therapeutic use of N-nonanoylvanillylamine contained in an orally consumable product, wherein the orally consumable product comprises no capsaicin, in a concentration 0.05 - 0.001 mg/kg based on the total mass of the orally consumable product for reducing the appetite and for imparting a feeling of fullness.

2. Method for the non-therapeutic reduction of the appetite and for the non-therapeutic mediation of a feeling of fullness, in which N-nonanoylvanillylamine contained in an orally consumable product, wherein the orally consumable product comprises no capsaicin, in a concentration 0.05 - 0.001 mg/kg based on the total mass of the orally consumable product is given to a human or animal subject.

3. Orally consumable product comprising N-nonanoylvanillylamine and one or more further substances, wherein
(i) the N-nonanoylvanillylamine is present in a concentration that reduces the appetite and/or brings about a feeling of fullness based on the total mass of the orally consumable product of 0.05 mg/kg or less and at the same time in a concentration of at least 0.001 mg/kg,
(ii) the orally consumable product comprises no capsaicin,
(iii) contains not more than 200 kcal/100 g of orally consumable product.

4. Orally consumable product according to claim 3, **characterized in that** the orally consumable product comprises, apart from N-nonanoylvanillylamine, no further capsaicinoids.

5. Orally consumable product according to at least one of claims 3 to 4, **characterized in that** the orally consumable product contains not more than 100 kcal/100 g of orally consumable product.

6. Orally consumable product according to at least one of claims 3 to 5, **characterized in that** the orally consumable product is selected from the group comprising confectionery, non-alcoholic drinks, instant drinks, cereal products, dairy products, products made from soy protein or other soybean fractions, sweetener preparations, sweetener tablets and sweetener sachets, ice-creams and dragees.

7. Orally consumable product according to at least one of claims 3 to 6, **characterized in that** the orally consumable product is yoghurt, kefir or quark.

8. Orally consumable product according to claim 7, **characterized in that** the orally consumable product comprises
(i) fruits and/or fruit preparations and/or
(ii) sugars and/or
(iii) thickeners and/or
(iv) gelling agents and/or
(v) sweeteners and/or
(vi) flavours and/or
(vii) preservatives.

9. Orally consumable product according to at least one of claims 6 to 8, **characterized in that** the orally consumable product comprises a probiotic.

10. Orally consumable product according to at least one of claims 3 to 5, **characterized in that** the orally consumable product comprises a chewing-gum base.

11. Orally consumable product according to at least one of claims 3 to 5, **characterized in that** the orally consumable product is a drink.

12. Preparation according to claim 3, **characterized in that** the preparation additionally comprises one or more TRPV1 inhibitors.

## Revendications

1. Utilisation non thérapeutique de N-nonanoylvanillylamine contenue dans un produit consommable par voie orale, qui ne contient pas de capsaïcine, en une concentration de 0,05 à 0,001 mg/kg, par rapport à la masse totale du produit consommable par voie orale, pour réduire l'appétit et pour procurer une sensation de satiété.

2. Procédé de réduction non thérapeutique de l'appétit et de procuration non thérapeutique d'une sensation de satiété, selon lequel de la N-nonanoylvanillylamine contenue dans un produit consommable par voie orale, qui ne contient pas de capsaïcine, en une concentration de 0,05 à 0,001 mg/kg, par rapport à la masse totale du produit consommable par voie orale, est administrée à un individuel humain ou animal.

3. Produit consommable par voie orale, comprenant de la N-nonanoylvanillylamine et une ou plusieurs autres substances, dans lequel
(i) la N-nonanoylvanillylamine est présente en une concentration réduisant l'appétit et/ou procurant une sensation de satiété, par rapport à la masse totale du produit consommable par voie orale, de 0,05 mg/kg ou moins et simultanément en une concentration d'au moins 0,001 mg/kg,
(ii) le produit consommable par voie orale ne comprend pas de capsaïcine,
(iii) et ne contient pas plus de 200 kcal/100 g de produit consommable par voie orale.

4. Produit consommable par voie orale selon la revendication 3, **caractérisé en ce qu'**il ne contient pas d'autres capsaïcinoïdes en plus de la N-nonanoylvanillylamine.

5. Produit consommable par voie orale selon au moins l'une quelconque des revendications 3 à 4, **caractérisé en ce qu'**il ne contient pas plus de 100 kcal/100 g de produit consommable par voie orale.

6. Produit consommable par voie orale selon au moins l'une quelconque des revendications 3 à 5, **caractérisé en ce qu'**il est choisi dans le groupe constitué par les confiseries, les boissons non alcoolisées, les boissons instantanées, les produits céréaliers, les produits laitiers, les produits à base de protéine de soja ou d'autres fractions de soja, les préparations édulcorantes, les tablettes édulcorantes et les sachets édulcorants, les crèmes glacées et les dragées.

7. Produit consommable par voie orale selon au moins l'une quelconque des revendications 3 à 6, **caractérisé en ce que** le produit consommable par voie orale est du yaourt, du kéfir ou du quark.

8. Produit consommable par voie orale selon la revendication 7, **caractérisé en ce qu'**il contient
(i) des fruits et/ou des préparations à base de fruits, et/ou
(ii) du sucre, et/ou
(iii) des épaississants, et/ou
(iv) des gélifiants, et/ou
(v) des édulcorants, et/ou
(vi) des arômes, et/ou
(vii) des conservateurs.

9. Produit consommable par voie orale selon au moins l'une quelconque des revendications 6 à 8, **caractérisé en ce qu'**il contient un probiotique.

10. Produit consommable par voie orale selon au moins l'une quelconque des revendications 3 à 5, **caractérisé en ce qu'**il contient une base de chewing-gum.

11. Produit consommable par voie orale selon au moins l'une quelconque des revendications 3 à 5, **caractérisé en ce qu'**il s'agit d'une boisson.

12. Préparations selon la revendication 3, **caractérisées en ce qu'**elles contiennent en outre un ou plusieurs inhibiteurs de TRPV1.
